(19) Europäisches Patentamt
European Patent Office
Office européen des brevets

(11) EP 4 507 501 B1

(12) **EUROPEAN PATENT SPECIFICATION**

(45) Date of publication and mention of the grant of the patent:
**18.02.2026 Bulletin 2026/08**

(21) Application number: **23723086.7**

(22) Date of filing: **13.04.2023**

(51) International Patent Classification (IPC):
***A23C 21/02*** *(2025.01)* ***C12P 7/56*** *(2006.01)*
***C22B 3/16*** *(2006.01)* ***C22B 3/44*** *(2006.01)*
***C22B 3/00*** *(2006.01)* ***C22B 11/00*** *(2006.01)*
***C22B 59/00*** *(2006.01)* ***C22B 7/00*** *(2006.01)*

(52) Cooperative Patent Classification (CPC):
**A23C 21/02; C12P 7/56; C22B 3/165; C22B 3/44; C22B 7/007; C22B 11/046; C22B 23/0415; C22B 23/0461; C22B 59/00;** Y02P 10/20

(86) International application number:
**PCT/IB2023/053795**

(87) International publication number:
**WO 2023/199263 (19.10.2023 Gazette 2023/42)**

(54) **PROCESS FOR PRODUCTION OF A LEACHING MIXTURE STARTING FROM DAIRY WASTE PRODUCTS**

VERFAHREN ZUR HERSTELLUNG EINER LAUGUNGSMISCHUNG AUS MILCHABFALLPRODUKTEN

PROCÉDÉ DE PRODUCTION D’UN MÉLANGE DE LIXIVIATION À PARTIR DE DÉCHETS DE PRODUITS LAITIERS

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **14.04.2022 IT 202200007502**

(43) Date of publication of application:
**19.02.2025 Bulletin 2025/08**

(73) Proprietor: **UNIVERSITA’ DEGLI STUDI DI CAGLIARI**
**09124 Cagliari (IT)**

(72) Inventors:
- **SERPE, Angela**
  **09124 Cagliari (CA) (IT)**
- **DE GIOANNIS, Giorgia**
  **09124 Cagliari (CA) (IT)**
- **MUNTONI, Aldo**
  **09124 Cagliari (CA) (IT)**
- **ASUNIS, Fabiano**
  **09124 Cagliari (CA) (IT)**
- **SPIGA, Daniela**
  **09124 Cagliari (CA) (IT)**
- **OUMAROU AMADOU, Amadou**
  **09124 Cagliari (CA) (IT)**
- **TRUDU, Stefano**
  **09124 Cagliari (CA) (IT)**
- **CERA, Martina**
  **09124 Cagliari (CA) (IT)**

(74) Representative: **Primiceri, Maria Vittoria et al**
**Praxi Intellectual Property S.p.A.**
**Via Leonida Bissolati, 20**
**00187 Roma (IT)**

(56) References cited:
**EP-A1- 3 950 977**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- ASUNIS F ET AL: "Control of fermentation duration and pH to orient biochemicals and biofuels production from cheese whey", BIORESOURCE TECHNOLOGY, ELSEVIER, AMSTERDAM, NL, vol. 289, 29 June 2019 (2019-06-29), XP085737326, ISSN: 0960-8524, [retrieved on 20190629], DOI: 10.1016/ J.BIORTECH.2019.121722

## Description

Technical field

The present invention refers to a process for the production of a leaching mixture or composition from waste of dairy products, more in particular the invention relates to a process for the treatment of raw waste of dairy products and dairy products with particular reference to the production of a leaching mixture or composition rich in lactic acid and lactates. More specifically, the waste is the liquid and fluid raw waste of dairy products such as whey and scald.

The invention is also relating to the use of the leaching composition obtained from the treatment of the waste of the dairy products indicated above in the areas of hydro-metallurgy and solvo-metallurgy for the recovery of metals from waste that contains them.

Figure 1 summarizes the object of this invention.

Background

Metals are widely used in traditional applications and new technologies. Due to their large and growing industrial use and the risks related to supplying natural resources, their recycling represents an irreplaceable activity to prevent the depletion of limited natural resources.

The recycling of metals often uses hydro-metallurgical processes based on the use of water based leaching products through dissolution, separation, purification and recovery of metal or metal compounds. These processes typically use strongly acidic or alkaline leaching solutions and/or the use of reagents highly dangerous for workers and environment.

On an industrial level, pyro-metallurgical methods are also used, thermal treatments aimed at the extraction and separation of metals, which are characterized by a high energy consumption and, based on the type of material treated, by harmful emissions.

Solvo-metallurgical processes are also currently developing, an example of which is described in Oumarou Amadou, A., De Gaudenzi, G.P., Marcheselli, G., Cara, S., Piredda, M., Spiga, D., Matharu, A.S., De Gioannis, G., Serpe, A. A New Easy Solvometallurgical Leaching Method for the Selective Co dissolution & Recovery from Hard Metals Waste (2021) International Journal of Refractory Metals and Hard Materials, 98, art. no. 105534. DOI: 10.1016/J.IJRMHM.2021.105534. The term "Solvo-Metallurgy" means the leaching and extraction of metals when using non-watery solutions, where not watery does not necessarily mean anhydrous, but a solvent with a low water content. The solvo-metallurgical processes are particularly useful when the leaching in water can interfere with materials and/or recycling processes. However, the implementation of these processes is often hindered by the cost and possible danger of the solvents used.

The patent document EP 3 950 977 A1 (Fundacion Tecnalia Res & Innovation [ES]) describes a process of hydro-and, potentially, solvo-metallurgical leraching applied to residues (in particular batteries, metal alloys and catalysts) containing metals (in particular Co, Ni and Mn) with complex mixtures containing lactic acid in the presence of protons acceptors, reducing agents, miscible with water organic solvents. The selected blends were in particular applied to the residues of cathods of lithium batteries with selective dissolution of said metals, present in the material in oxidized form, and their partial recovery from the solutions by precipitation. The case of HDS catalysts containing Al, Co and Mo treated in order to recover Co is described. The document does not detail, however, the specific operating conditions and the results relating to the treatment of metals other than those mentioned, of other materials wherein the metals are present in a reduced form and of most of the leaching mixtures described. The reagents reported in EP 3 950 977 A1 are of commercial degree and are suitably mixed according to the recipes described. In most cases the mixture described includes ingredients, including choline chloride, characterized by a high cost. Otherwise, the patent document EP 3 950 977 A1 does not describe the application of leaching mixtures obtained as a direct result of fermentative processes for the enhancement of matrices and/or organic waste (therefore consisting of a mixture, naturally derived from the fermentative process, of organic acids and/or proteins and/or fats, in variable proportions).

As regards the origin of the lactic acid that can be used as a leaching agent, globally 90% of the lactic acid is currently produced by microbial fermentation starting from pure carbohydrates (e.g. glucose, lactose) or contained in others substrates, also waste (sugar beet, molasse, whey and malt) (Ghaffar et al., 2014 Journal of Radiation Research and Applied Sciences, 7, 222-229, http://dx.doi.org/10.1016/j .jrras. 2013.03,002).

When considering only the production of lactic acid from whey and scald deriving from the dairy industry, at present, there is no economically advantageous and consolidated process on industrial scale.

The possibility of producing lactic acid from whey and scald was investigated in some studies performed on a laboratory scale, the processes were fermentative type in anaerobic conditions and in the absence of light. In particular, in the study of Liu et al. (2018) (Process Biochemistry, 71, 18-22, https://doi.org/10.1016/j.procbio.2018.05.014) is obtained an average concentration of lactic acid equal to 113.18 $gL^{-1}$ starting from whey in powder form, using an inoculum

consisting of *L. Bulgaricus* CGMCC 1.6970, with a 42°C Fed-Batch process management strategy and adding an enzyme (alkaline protease) in order to make whey proteins available and 45 $gL^{-1}$ of CaCOs to maintain the pH with values equal to about 5.5. In Bernando et al study (2019) (Brazilian Journal of microbiology, 47, 640-646, http://dx.doi.org/10.1016/j.bjm.2015.12.001) a concentration of lactic acid equal to 143.70 $gL^{-1}$, always starting from whey powder by adding an external inoculum (*L. Rhamnosus* B103) and a co-substrate (Corn Steep Liquor, or "wheat liqueur"), with a process management of the "Fed-Batch" type. In recent years, it has also been tried to develop the biological production of lactic acid from species other than bacteria, such as in Turner's et al. (2017) (Journal of Dairy Science, 100, 1, 124-128) wherein it is proposed to use an engineered yeast (*S. Cerevisiae*) for the fermentation of cattle whey, however obtaining a concentration lower than those previously reported and equal to 10.3 $gL^{-1}$.

It should be emphasized that these methodologies, implemented only on a laboratory scale, make use of powder whey (and therefore need a strong drying step of the whey as it is) and an external inoculum (with costs related to the supply of the bacterial inoculum and the need to keep the reaction environment sterile). About the use of external inoculum, Juodeikiene et al. (2016) (LWT-Food Science and Technology, 72, 399-406) proposes the use of an acid-resistant bacterial strain (*Pedioccocus SPP.*) to limit the use of chemical reagents necessary for the neutralization of the produced lactic acid, obtaining a final lactic acid concentration in the range 47-57 $gL^{-1}$. In fact, during lactic fermentation, pH tends spontaneously to decrease until it reaches the pKa value of lactic acid (3.83), a value that has an inhibiting effect against lactic microorganisms present, including lactobacilli that do not tolerate pH <4 (Current Limitions and Challenges with Lactic Acid Bacteria: A review Saeed A. Hayek, Salam A. Ibrahim Food & Nutritional Sciences Program, North Carolina Agricultural and Technical State University, Greensboro, USA DOI:10.4236/fns.2013.411A010). This makes continuous control of the pH necessary with the addition of appropriate base quantities.

The patent document NZ 287 397 A (Fraunhofer Ges Forschung) relates to a multistep purification process of liquid dairy waste where lactic fermentation represents one of the treatment steps in order to break down the initial organic load. The fermentation is induced on a previously highly alkalized waste (pH> 7-10) to induce calcium, magnesium and phosphates precipitation, and is promoted by an external inoculum of lactobacilli checking the pH to values > 6. The lactates produced by the conversion of lactose are concentrated and separated via chemical-physical ways from the purified waste. The saline fraction is then subjected to electrodialysis with the production of lactic acid. Although the process provides for a fermentation step, the production of associated lactic acid is neither controlled nor oriented towards specific industrial applications, therefore it is not optimized in the sense of obtaining a technically, environmentally and economically sustainable enhancement process, wanting rather to obtain the waste purification. For this reason, in the aforementioned document, reagents, inocula and pretreatment steps, concentration and separation which, if applied to a process for enhancing the waste, would make it uneconomical and not very efficient, is widely used.

The fermentation processes applied to whey/scald are, in fact, currently considered particularly attractive on an industrial scale, mainly due to the low concentration of convertible carbohydrates which is much less favorable than that of other natural substrates that are typically used for the purpose, as well as for the costs associated with the control of the process pH which requires strong addition of reagents, and the use of specific inocula designed to promote the conversion of carbohydrates into lactic acid.

It is known that the fermentation of biomass containing high percentages of carbohydrates may be, in appropriate conditions, a valid system for producing organic acids, including lactic acid. However, a relevant point to be underlined is that the fermentation mediated by unselected bacteria, indigenous of the raw whey/scald, although spontaneous and low cost, can evolve towards the conversion of lactic acid produced in other organic acids (acetic, butyric and/or propionic acid), gaseous hydrogen and carbon dioxide according to some well -known metabolic reactions. In fact, there is currently no sustainable method (in terms of costs and use of reagents) which inhibits the action of bacteria that consume lactic acid, while guaranteeing the maximization of the conversion of lactose in lactic acid. The maintenance of an acid pH (e.g. pH = 5.5) is not effective as demonstrated in Asunis et al., 2019, while an even more acidic pH (<5) limits the complete conversion of lactose into lactic acid (see figure 2b; Asunis et al., 2019 https://doi.org/10.1016/j.biortech.2019.121722).

For these reasons, currently the cited dairy waste is mainly treated in purification plants without any enhancement or, in some cases, used as an animal feed and therefore valued, but obtaining a very low profit.

In practice, all the documents of the known technique tend to maximize the consumption of carbohydrates in order to lower the organic load (COD) in order to dispose of the waste more easily or at most recover lactic acid and lactates at the end of fermentation through additional separation methods.

The invention aims to solve the problems connected with the use of liquid waste of the dairy industry, in particular whey and scald, through the development of a process based on the control of anaerobic fermentation in the absence of light (Dark fermentation, DF):

- in order to obtain the production of low-cost leaching mixtures, containing organic acids or their salts that are effective on a wide variety of metals;
- in the application of these leaching mixtures for the hydro- solvo-metallurgical recovery of critical metals from

industrial waste and scrap, including those of the hard metal supply chain, with particular reference to the recovery of Co, and the waste from electrical and electronic equipment (WEEE).

If not specifically excluded in the detailed description that follows, what is described in this chapter is to be considered as an integral part of the detailed description of the invention.

Summary of the invention

A purpose of this invention is therefore to provide a method or process for the controlled treatment of liquid or fluid waste of dairy products, in particular the whey and scald as claimed in claim 1 in order to maximize the obtaining of lactic acid and lactates to be used as leaching agents with minimum or even without pH adjustments.

Another purpose of the invention is the use of the mixture or composition obtained with the process for the controlled treatment of liquid or fluid waste of dairy products, in particular whey and scald, as a leaching agent for the treatment of metals and waste metal materials.

These and other purposes, advantages and characteristics of this invention will be better specified in a detailed description of the preferred embodiments that follows, moreover the claims describe preferred variants of the invention, forming an integral part of this description.

Brief description of the figures

The purposes and advantages of this invention will be clear from the detailed description that follows, an example of realization of the same (and its variants) and by the attached drawings, provided for pure explanatory and non -limiting scope, wherein:

- Fig. 1. schematically illustrates the various steps of the invention process;
- Fig. 2 illustrates in the chart: (a) the trend of the concentration of carbohydrates and lactic acid during fermentation and (b) the trend of the concentration of lactic acid and other organic acids in the fermentation at pH 6;
- Fig. 3 illustrates the trend of the molar fractions of lactic acid and carbohydrates soluble at the time of the $k^{th}$ sample and the pH value obtained at the end of fermentation in correspondence with the $q_k$ quotient, in the absence of pH check;
- Fig. 4 illustrates in a picture the fermented milk whey according to the invention after drying (a) and subsequent extraction with EtOH (b);
- Fig. 5 illustrates in chart the multistep leaching of the shredded WEEE (1 g) with fermented whey after purification (PFW) (pH 4). Chart (a1): leaching profile (metal mg leached over time)*; Conditions: 1st step: 8 h, 200 ml leaching agent, $T_{amb}$; 2nd step: 8 h, 200 ml leaching agent, Δ; 3rd step: 48 h, 200 ml leaching agent, $H_2O_2$, Δ; 4th step: 24 h, 50 ml leaching agent, $H_2O_2$, Δ; chart (b1): distribution of the leached metal (%) between the leaching solutions obtained from the set of conditions of a1. Chart (a2): leaching profile (leached metal mg over time)*; Conditions: 1st step: 8 h, 200 ml leaching agent, $T_{amb}$; 2nd step: 8 h, 200 ml leaching agent, Δ; 3rd step: 48 h, 200 ml leaching agent, $H_2O_2$, $T_{amb}$ ; 4th step: 24 h, 50 ml leaching agent, $H_2O_2$, $T_{amb}$ ; chart (b2): distribution of the leached metal (%) among the leaching solutions obtained from the set of conditions a2. *2nd, 3rd and 4th step, cumulative masses of each metal with the solutions of the previous steps;
- Fig. 6 illustrates the % metal composition of the 1, 2, 3, and 4 solutions in chart deriving from the leaching steps with PFW pH 4 0.5 M. a: 1 ($T_{amb}$), 2 (Δ), 3 and 4 (Δ $H_2O_2$) ; b: 1 ($T_{amb}$), 2 (Δ), 3 and 4 ($T_{amb}$, $H_2O_2$);
- Fig. 7 illustrates in chart the results of the leaching on shredded RAM cards with PFW and commercial HLat at room temperature (A) and at 100°C in the microwave oven (B) both in concentration 0.5M, L/S = 50.
- Fig. 8 illustrates a magnet as extracted from a hard-disk (a) and after treatment at 200°C with (b) and without (c) Ni coating.

Detailed description of the invention

Within the aim of the present invention with the wording "dairy waste" or "raw dairy waste" is meant the raw

residue, substantially liquid or fluid deriving from the processing of milk aimed at the production of dairy products. In the wording "dairy waste" or "raw dairy waste", the milk whey is included, which is the main by-product of the cheese production process.

Within the aim of the present invention, the wording "milk whey" means: liquid residue obtained following the production of cheeses. The milk whey can derive from cattle, sheep, goat, buffalo and any other animal source from which dairy products are obtained.

Within the aim of the present invention with the wording "scald", is meant the liquid residue obtained following the production of ricotta cheese by appropriately processing the whey.

Within the aim of the present invention, the leaching compositions obtainable with the methods and processes described below may be indicated, regardless of each other, in the physical forms of "mixtures", "solutions", "suspensions", "creams", "fluids", therefore all said physical forms will be attributable to the wording "composition(s) leaching agent(s)" or "leaching agent(s) product".

Within the aim of the present invention with the wording "fermented whey after purification" or "PFW" is meant the mixture obtained from the fermentation of whey or scald according to the invention that has been subjected to purification by precipitation of an insoluble organic substance following addition of alcohol or acetone to the fermented solution in ambient conditions as already known by consolidated processes (https://www.journalofdaIRYSCIENCE.org/article/s0022-0302(70)86362-7/pdf).

Within the aim of the present invention with the wording "hard metal" (or "cemented carbide") is meant a material consisting of tungsten carbide hard particles (WC) incorporated into a metal matrix.

Within the aim of the present invention, the wording "about" means a numerical value of $\pm$ 0.5.

Within the aim of the present invention, the wording "liquid/solid ratio" or "L/S" indicates the ratio between the volume expressed in liters of leaching mixture and the mass expressed in kg of the solid treated sample.

Within the aim of the present invention with the "base" wording, are meant one or more compounds chosen between, but not limited to, inorganic bases including alkaline/alkaline-earth, carbonates, and corresponding mixtures, for example in solid form or as concentrated aqueous solution (3-10M), or buffer systems such as ammonia, phosphate, borate.

Within the aim of the present invention, the "lactic acid" wording is intended to include all forms of lactic acid, i.e. D- and L- lactic acid in protonated and not protonated form. The not protonated lactic acid is also called lactate ion and can be found in saline or coordinated form. Therefore, the lactic acid wording is also intended to include the forms of D-lactic acid and salts/complexes thereof, L-lactic acid and salts/complexes thereof. In the following it is possible also to find the lactic acid/lactate wording with the same meaning indicated here.

The concentration of lactic acid can be determined with numerous well-known methods to an expert in the field, such as measures through HPLC (High Performance Liquid Chromatography), UV-Vis spectrophotometry, enzymatic essays for example based on D/L-Lactate dehydrogenase or nuclear magnetic resonance imaging (NMR).

Within the aim of the present invention, the wording "carbohydrates" is intended to include the soluble carbohydrates contained in the whey/scald, mainly represented by simple carbohydrates (sugars) of which the prevalent is lactose.

The concentration of carbohydrates can be determined with numerous well -known methods to an expert in the field, such as the refractometry coupled to HPLC, for example according to the official method 984.12 of the association of analytical communities (AOAC); Polarimetry coupled to HPLC; or enzymatic essays, for example based on the enzymatic hydrolysis of glucose and galactose lactose at pH 6.6 by $\beta$-galattosidase, subsequent oxidation of $\beta$-galactose released to galactonic acid to pH 8.6 $\beta$-galactose dehydrogenase, then followed by reduction of the Nicotinammide Adenina Dinucleotide (NAD+) whose concentration is determined by reading 340 nm absorbance, in particular according to the official AOAC 984.15 method; or by means of a phenol/sulfuric acid colorimetric method with spectrophotometric determination of absorbance at 490 nm [Dubois M, Gilles K.A., Hamilton J.K., Rebers P.A., Smith F., Colorimetric Method for Determination of Sugar and Related Substances, Division of Biochemist, University of Minnesota, https://ediscipisci nas.usp.br/pluginfile.php/5012807/mod resource/content/0/dubois 1956%20%28m%C3%A9todo%29. pdf].

This invention relates to:

the production of bio-derived leaching mixtures or compositions, sustainable and low cost for the selective leaching of the metals, capable of replacing the use of commercial reagents and enhancing a dairy waste that currently constitutes a waste to be treated, and

the direct application of these leaching mixtures or compositions in the dissolution of metals for their recovery from raw and secondary materials.

The fermentation conditions are modulated thanks to a specific process monitoring wherein they measure:

the molar concentration at the start and during the fermentation of the carbohydrates performed on the raw waste (liquids or fluids) deriving from the dairy production;

the molar concentrations of lactic acid and lactates in the same time intervals.

Fermentation is a fermentation in the dark (DF) in anaerobic conditions and is performed on the raw waste (liquid or fluid) deriving from the dairy production without any addition of inoculum of biomass from the outside. The vast scale implementation of the processing treatment according to the invention is based on the indigenous mixed microbial cultures and/or on the lactic bacteria (LAB) that are found in dairy waste and involves various advantages, for example no need for sterilization of the substrate, no additional cost for the dedicated inoculum, no energy consumption for the pretreatment of the substrate, thus making the raw waste a particularly attractive substrate from the point of view of the circular economy and allowing to implement the process in a simple way.

The control of fermentation takes place through monitoring the $q_k$ parameter defined as:

$$q_k = [HLat+lactates]_k/[carbo]_k$$

wherein:

- $[HLat+lactates]_k$ is the molar concentration of lactic acid+lactates at the time of the withdrawal k, measured with at least 2 significant digits,
- $[carbo]_k$ is the molar concentration of the liquid waste carbohydrates at the time of the withdrawal k, measured with at least 2 significant digits,
- k = positive integer greater than 0, k is a discreet quantity associated with the number of subsequent withdrawals distant over time of at least 30 minutes;

In particular in raw waste (containing families of bacteria, lactic and not, indigenous and not, controlled by sterilization and specific inoculum), this parameter has a growing trend ($q_{k-1}<q_k$) as long as lactic acid/lactate is produced and not consumed, while it has a decreasing trend ($q_k>q_{k+1}$) when, at the end of the conversion of carbohydrates in lactic acid for homolactic fermentation by the lactobacilli present (indicated by the achievement of about 95% or higher of the carbohydrates converted into lactates), lactic acid starts being converted, through acidogenic fermentation, in other organic acids, gaseous hydrogen and carbon dioxide by acidogenic bacteria (see figures 2a and 2b).

The process or method to produce a leaching mixture or composition from raw waste of dairy products according to the invention is conducted in the aqueous liquid phase in anaerobic conditions and in the absence of light, it comprises the following steps:

- -performing a fermentation in liquid phase in the absence of light and in anaerobic conditions on raw waste, checking the pH by adding a base so that this is maintained in the interval of about 4.5-6.5, preferably about 5.0-6.0; particularly preferred is the interval about 5.5-6.0 or the pH is maintained at about 4.5, about 5.0, about 5.5, about <6.0; the fermentation temperature is typically ≤ 40°C, preferably of the order of 25-39°C, more preferably 35-39°C, temperature that can be advantageously that of the raw whey leaving the processes traditionally performed inside the dairy;
- checking the fermentation by performing withdrawals to determine the molar concentration of lactic acid and lactates as well as the molar concentration of carbohydrates in the fermentation mixture;
- continuing the fermentation in appropriate experimental conditions detailed below until the end of the conversion of carbohydrates into lactates (indicated by the achievement of about 95% or higher of the carbohydrates converted into lactates) highlighted by the $q_k$ parameter in correspondence with the first set of $k^{th}$ withdrawals that verify the following condition:

$$q_{k+2} \leq q_{k+1} \leq q_k$$

- recovering the leaching solution by separation from the reaction biomass, the recovery can be done simply by sedimentation;
- optionally it is possible to add a purification step to eliminate proteins and impurities from the leaching solutions

obtained, this step can be advantageously performed by adding alcohol or acetone to the fermented solution at ambient conditions as already known by consolidated processes (https://www.Journalofdalryscience.org/article/s0022-0302(70)86362-7/pdf);

- the purification step can be followed or replaced by a drying or dehydration step to obtain the product in dusty form; drying can be performed with any of the traditional methods such as evaporation of water in a stove or by spray-dryer or by freeze drying or any other method of eliminating the water known in itself.

The fermentation conditions are modulated thanks to a specific process monitoring in order to obtain leaching solutions with the characteristics of [HLat] and pH most suitable for the type of material to be subjected to leaching.

The $q_k$ parameter chosen and identified above is particularly advantageous as it is independent of time, a parameter that proved to be unreliable for the correct control of the process, as well as the type of raw waste chosen (origin of the milk: sheep, goat, vaccine, buffalo, etc.) and its conservation methods (such as: refrigeration, freezing, freeze-drying) and reconstitution (such as: rehydration, thawing).

For example, if a previously frozen and thawed raw waste such as whey or scald is used, these treatments do not inhibit the managing of the process according to the invention. In case an in advance lyophilized/dried/dehydrated raw waste such as whey or scald is used, this can be advantageously dissolved in water (rehydrated), or mixed with raw whey just produced or thawed in any desired proportion, or rehydrated and added to residual biomass deriving from previous fermentation processes of the raw waste described.

The method or process of the invention can be advantageously placed in line with the typical dairy production processes, as the whey is in the optimal thermal conditions to start the fermentative process of the invention on site, limiting the economic and environmental costs linked to the raw whey storage step (such as drying, refrigeration or freezing), pre-fermentation and restoration of the chemical-physical and thermal conditions necessary for fermentation itself.

According to a preferred embodiment, the addition of the base can be continued until the end of the conversion of carbohydrates into lactates (indicated by the achievement of about 95% or higher of the carbohydrates converted into lactates, see Figure 2a, said conversion being highlighted by $q_k$ parameter in correspondence with the first set of $k^{th}$ withdrawals that verify the following condition $q_{k+2} \leq q_{k+1} \leq q_k$) obtaining **Mixture A** (characterized by a pH about 6). In the processes of the known art performed at pH about 6.0-6.5 the moment wherein the conversion of carbohydrates in lactates ends and the consumption of lactates in favor of the concomitant acidogenic reactions begins is not determined.

The identification of the moment wherein the conversion of the carbohydrates in lactates ends is of fundamental importance since, as mentioned above, in these conditions it has been shown that lactic acid/lactate is a substrate for other families of indigenous microorganisms capable of converting it in other organic acids, hydrogen and carbon dioxide as described by the chart in figure 2b.

According to a further preferred embodiment of the invention, the addition of the base can be interrupted at a certain point so that the bacteria continue to work spontaneously up to the consumption of carbohydrates present in the fermentation environment with consequent decrease of pH below about 6. This embodiment is aimed at obtaining a leaching agent acidic mixture **(Mixture B,** pH $\leq$ 4) characterized by the maximum conversion of carbohydrates in lactic acid minimizing the use of chemical control agents for pH through the acidifying action of lactic bacteria.

The most suitable time for the interruption of the base addition is identified by achieving a specific value of $q_k$, where $q_k$ = $[HLat+lactates]_k/[carbo]_k$ as previously defined. In case obtaining a leaching agent acid mixture is intended, this ratio will be between about 4 and about 5. The concentration of lactic acid/lactate will be advantageously monitored and measured instant by instant during the anaerobic fermentation process. When the ratio reaches around 4 and 5, the delivery of the base used (and the relative monitoring of the $q_k$ control parameter) can be interrupted to maintain pH around 6, because from that point on in the fermentation reactor there will be carbohydrates in necessary and sufficient quantities so that their transformation/metabolization by bacteria spontaneously drops the pH to the desired final value (pH $\cong$ 4, corresponding to the pH of the lactic acid /lactate buffer solution). From this procedure a mixture called **Mixture B** (characterized by pH $\leq$ 4) is obtained.

Advantageously, the $q_k$ = $[HLat+lactates]_k/[carbo]_k$ ratio identified above is a parameter independent of the fermentation lasting (t) and of the chemical-physical characteristics (including pH, composition of nutrients, quantity of initial carbohydrates) of the whey/ scald input.

The method for obtaining **Mixture A,** characterized by a pH about 6 and by a content of negligible (or even absent) lactic acid compared to lactates,

*or*

the method for obtaining **Mixture B,** characterized by pH $\leq$ 4 and by a content of [HLat]/[lactates] 1:1, comprises:

- fermenting at pH $\cong$ 6, the liquid or fluid waste of the dairy industry preferably chosen from milk whey and scald, in anaerobic conditions in a reactor closed and equipped with an automatic pH control system, at atmospheric pressure, in the absence of light, preferably under stirring (for example: 120-150 rpm), at a temperature lower than 40°C,

preferably 35-39°C; the liquid or fluid waste can be taken as it is from the end of the dairy processes, it can be thawed or obtained by rehydration from whey/scald powder;

- quantifying initial carbohydrates in terms of molar concentration;
- feeding in the reactor amounts of a base to keep pH around 6;
- continuing fermentation in the conditions described above (in the dark, under stirring, at atmospheric pressure and temperature lower than 40°C, preferably 35-39°C) always maintaining pH around 6, until the end of the conversion of carbohydrates in lactic acid, said conversion being highlighted by the $q_k$ parameter in correspondence with the first set of $k^{th}$ withdrawals that verify the following condition $q_{k+2} \leq q_{k+1} \leq q_k$ and before the lactic acid/lactate produced starts being converted into other organic acids, hydrogen and carbon dioxide, if a leaching agent wherein lactic acid is completely deprotonated is desired,
*or*
- monitoring instant by instant, the reference parameter $q_k$ = [HLat+lactates]$_k$/[carbo]$_k$ and when it stands between about 4 and about 5 stopping the base addition and letting the reaction spontaneously continue, if a leaching agent with an acid pH is desired. With reference to the final step of the base addition to obtain the leaching agent with an acid pH, it will be advisable to carefully verify it as (see Figure 3):
    - If the base addition is interrupted at a too early step ($q_k$ = [HLat+lactates]$_k$/[carbo]$_k$ <4), the bacterial fermentation of the residual carbohydrates leads to reach the desired pH (3.8-4) before the present carbohydrates are completely converted, with the consequent inhibition of the fermentative activity of the bacteria: this involves the incomplete conversion of carbohydrates and a low concentration of lactic acid/lactate in the leaching solution;
    - If the base addition is interrupted at a too late step ($q_k$ = [HLat+lactates]$_k$/[carbo]$_k$ >5), the residual carbohydrates are completely converted to lactic acid/lactate by bacterial fermentation, but in concentration insufficient to achieve the desired pH: the leaching solution will have the maximum obtainable lactic acid/lactate amount, but pH will not be the desired one (final pH> 4).

At the end, recovering the leaching agent solution by separation from the reaction biomass; the recovery can be made simply by sedimentation.

Optionally, a purification step can be added to eliminate proteins and impurities from the leaching solutions obtained, this step can be advantageously carried out by adding alcohol or acetone to the fermented solution at ambient conditions as already known by consolidated processes (https: //www.journalofdairynscience.org/articles/S0022-0302 (70) 86362-7/pdf).

The purification step can be followed or replaced by a drying/dehydration step to obtain the product in a powder form.

Depending on the methods of controlling the fermentation as indicated above, it is possible to obtain a range of mixtures based on lactic acid/lactate which are all variously used as leaching agents (both in water and in another solvent). In fact, it is possible to get a mixture **(Mixture B)** with a [HLat]/[lactate] 1:1 ratio characterized by a pH around 3.8-4.0 which is the buffering pH or it is possible to get a mixture **(Mixture A** with 100% lactate) wherein lactic acid is practically absent and by appropriately regulating the reaction conditions it is possible to obtain all intermediate ratios. The expert in the field, with his knowledge and reading the teachings of this invention, will be able to easily obtain the best mixture for uses as a leaching composition described here below.

The mixture obtained with the process according to the invention can be used as such, after separation for decanting of solid biomass, as a leaching solution or the mixture obtained can be treated with alcohol, for example ethanol to purify it of the protein part, according to methods known in the art.

In order to improve and facilitate its conservation and storage, as well as to obtain higher concentration leaching solutions, the mixture obtained with the invention process can be subjected to drying/dehydration (for example by evaporating water in a stove or by spray drying or by freeze drying or other known method of eliminating water). The dried product can subsequently be reconstituted in water and/or alcohols to the desired concentration and used as a leaching solution.

The use of the mixture as a leaching solution is particularly advantageous as its performances are similar or superior (see examples) compared to those of the commercial lactic acid and its derived solutions with different pH, with the advantage of the enhancement of a waste that, otherwise, should be disposed of with economic and environmental costs.

In addition, as part of the use of the mixtures having pH about 5.5-6, the total deprotonation of lactic acid to form almost quantitatively the lactate ion maximizes the complexing capabilities of the mixture and makes it particularly suitable

for the solvo-leaching and/or for the treatment of materials containing oxidized metals.

To summarize, the liquid mixture or the dehydrated/dried product or in fluid form (or cream) obtained according to the invention can be used in the field of hydro-metallurgical and solvo-metallurgical leaching:

- ◦ as such; or
- ◦ It can be purified (for example by filtration) by separating the possible precipitated protein portion as indicated above. The possible protein portion can be enhanced mainly as a nutraceutical product;
- ◦If an increase of the concentration of lactic acid /lactate is desired, it is possible to dry the mixture, and the fermented whey powder can then be redissolved in water or alcohol, alone or in mixture, until the desired concentration.

The solutions/mixtures/compositions obtained as such from the process of invention, the aforementioned fluid forms or the powders or the solutions/mixtures/compositions reconstituted have proven extremely efficient in ambient conditions in the selective dissolution and recovery of metals, in particular of Co from hard metal matrices of WC, as well as base metals such as for example, but not limiting itself to, Co, Pb, Ni, Zn, Sn, Al, and some noble metals (such as Cu, Ag) from matrices consisting of heterogeneous multi-metal mixtures obtained for WEEE's shredding, as well as base metals (Fe, Co) and lantanoids (or "Rare earths", Nd, Dy, Pr) from permanent magnets such as those based on the $Nd_2Fe_{14}B$ alloy, present in elementary or oxidized form by properly directing the fermentation and the leaching operating conditions.

By means of this invention it is possible to enhance the waste:

- from the dairy sector, which are currently mainly considered products to be disposed of, with related economic and environmental costs;
- from the production sector of the hard metal, wherein the appropriate modulation of the composition of the regenerated composite materials (relative quality and quantities of the components, WC and Co) is a crucial aspect for the industrial reuse of the waste;
- from electrical and electronic equipment (WEEE), for sustainable management of end of life materials that prevents the formation of polluting waste and limits the use of the supply of virgin raw materials.

The method or process of the invention as described above allows to obtain leaching compositions with characteristics of concentration of lactic acid/lactates controlled in a pH range 3.8 to 6.5, in particular mixtures/compositions substantially based on lactates and substantial absence of lactic acid (pH≈6, **Mixture A),** or mixtures/compositions about 1:1 of lactic acid - lactate (pH ≤ 4, **Mixture B)** through the fundamental steps of:

A) Lactic fermentation of the liquid or fluid waste of the dairy industry, preferably chosen from milk whey and scald, in anaerobic conditions in a closed reactor equipped with a pH automatic control system, at atmospheric pressure, in the absence of light, under stirring (for example 120-150 rpm), at a temperature lower than 40°C, preferably 35-39°C, monitored and controlled according to the $q_k$ = $[HLat+lactates]_k/[carbo]_k$ process ratio obtaining:

**Mixture A:** pH control to about 6 with the addition of a base such as NaOH, in order to guarantee the bacteria present in the waste a reaction environment constantly suitable for their survival until the end of the conversion of carbohydrates into lactates and interrupted before further conversion of lactates to other organic species wherein the balance:

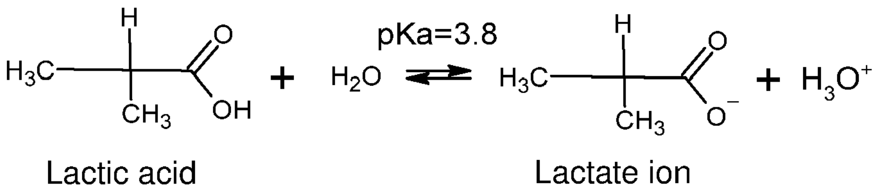


it is completely shifted towards the presence of the lactates only;
*or*

**Mixture B:** pH control to about 6 with the addition of a base such as NaOH until reacing the value $q_k$ = $[HLat+lactates]_k/[carbo]_k$ between about 4 and about 5; fermentation is then made to continue in the absence of

pH control with the production of lactic acid/lactate mixtures about 1:1 until substrate consumption and achievement of a pH that inhibits bacteria. In this step pH drops spontaneously following the presence of the lactic acid produced by fermentation and not neutralized by the addition of the base (leaching Mixture B at pH ≤4, lactic acid: lactate ratio about 1:1).

B) then follows (optionally) the removal of potential organic interferents (bacterial biomass and proteins) by simple chemical-phisical separation (decantation, separation) possibly assisted by the addition of recyclable solvent (ethanol/acetone) through known methodologies.

C) the mixtures/compositions obtained with the invention process can be used in the treatment of metals or materials containing metals by selective leaching, appropriately choosing the necessary characteristics, as summarized in the following table which is to be considered illustrative and not limiting:

<table>
<tr><th>MIXTURE</th><th>Solvent</th><th>External oxidant[a]</th><th>Metallic leachable species</th></tr>
<tr><td rowspan="4">A</td><td>$H_2O$</td><td rowspan="2">NO</td><td rowspan="2">Metal oxides[b]</td></tr>
<tr><td>Organic[e]</td></tr>
<tr><td>$H_2O$</td><td rowspan="2">YES</td><td rowspan="2">Base oxides[c]<br>Noble metals[d]<br>Metal oxides[b]</td></tr>
<tr><td>Organic[e]</td></tr>
<tr><td rowspan="4">B</td><td>$H_2O$</td><td rowspan="2">NO</td><td>Base oxides[c]<br>Metal oxides[b]</td></tr>
<tr><td>Organic[e]</td><td>Metal oxides[b]</td></tr>
<tr><td>$H_2O$</td><td rowspan="2">YES</td><td rowspan="2">Base oxides[c]<br>Noble metals[d]<br>Metal oxides[b]</td></tr>
<tr><td>Organic[e]</td></tr>
<tr><td colspan="4">[a] examples of oxidants used, not limited to:: $O_2$, $H_2O_2$, $I_2$, $Fe^{3+}$, $Cu^{2+}$, $O_3$, etc. The selection of oxidant affects the selectivity of the leaching;<br>[b] pure or mixed oxides of metals and metals in oxidized form (e.g. in saline compounds);<br>[c] elementary metals characterized by E°<0, including rare earths, also constituting alloys;<br>[d] metals in the elementary state characterized by E°> 0;<br>[e] typically, but not exclusively, alcohols, acetone, methytylchetone, ether and other organic solvents wherein lactic acid and lactate salts are soluble.</td></tr>
</table>

The invention is focused on the use of the leaching agent composition of the invention in selective leaching and recovery of often critical and/or toxic metals including Co, Ni, Pb, Zn, contained in industrial waste including WEEE and "hard metals" using the lactic acid-based solutions obtained as a product of the controlled biological fermentation of residues of the dairy industry.

More specifically:

- **Selective leaching of Co from WC-Co waste powders from the "hard metal" chain.** The simplest case of study provides (by comparison) the use of commercial lactic acid (FU-BP E270, ≥80%) in aqueous solutions or non-aqueous solutions, that is to say based on organic solvents wherein lactic acid and lactates are soluble, for example methanol, ethanol, isopropanol and other alcohols; acetone and other superior compounds; alkylacetates; hydrocarbons; etc.) for the treatment of hard metal powder with WC-Co composition (obtained, for example, as a waste of the production process or from regeneration of the hard metal scrap for the purposes of recycling materials). Experimental results have shown that aqueous lactic acid solutions with 0.1-1M concentration, pH ≤ 4, under stirring at room temperature and atmospheric pressure, in appropriate ventilation conditions, selectively and efficiently dissolve (up to quantitative dissolution) the cobalt contained in the powder in a few hours. Using the Co selective leaching from the above powders with the fermented whey and scald at pH ≤ 4 in leaching conditions (solvent, temperature, pressure, ventilation, L/S ratio) formerly applied by using commercial lactic acid, the efficiency and the time of Co dissolution are demonstrated comparable with those obtained by using the leaching agent compositions of the invention, thus guaranteeing the full usability of this fermented diary waste for this application.

A further case of study provides for the selective leaching of Co (quantitative or partial according to the final composition required for recycled powder based on WC) from the mentioned hard metal powders, using the fermented whey and scald at pH = 6 in organic solution (in this case, the fermented whey/scald obtained at pH = 6 had been dried and solubilized in organic solvent, e.g. ethanol) in the presence of iodine as a recyclable oxidizing agent at room temperature, under stirring.

The leaching step is then followed by the enhancement of: i) the leaching solution through the recovery of the $[Co(Lat)_2(H_2O)_2]$ complex as a precursor of metallic Co, Co oxides or carbonates to be reused for production purposes or by application in catalysis and livestock nutrition; ii) enhancement of the WC recovered for production purposes;

- **Selective leaching of base and noble metals from WEEE.** The case study focuses on a multimetallic material obtained by shredding small electronic equipment. The experimental results have shown that commercial lactic acid solutions at different pHs, including pH 4, with varying temperatures and in the absence and presence of external oxidizing agents ($O_2$, $H_2O_2$) are capable of selectively leaching metals, or groups of metals. The use of the "model" or "comparison" leaching solution has made it possible, in particular, to obtain a selective leaching of the base metals (reductive potential < 0, with the exception of nickel) already at ambient conditions, an increase of the efficiency towards iron and nickel as the temperature increases (reflux), and a dissolution of copper and silver by addition of $H_2O_2$. The use of fermented whey as a leaching agent confirmed the good results obtained with the model solutions and highlighted a greater leaching efficiency on iron and nickel in ambient and hot conditions which allowed a quantitative dissolution of said two metals. A non-harmful effect of the organic substance residues contained in the mixture of the invention has also been demonstrated on the recovery steps and on the recovered materials. These selective leaching steps are of fundamental importance for the downstream recovery of the precious metals which remain unreacted.

- **Dissolution of base metals and "rare earths" from permanent magnets.** The case study focuses on the low environmental impact leaching of metal alloys that make up the $Nd_2Fe_{14}B$ permanent magnets and/or the related metal oxides resulting from thermal pretreatments, using **Mixture B** at pH $\leq$ 4. The use of Fermented Whey after Purification (PFW) on thermally pretreated hard-disk permanent magnets led to the complete dissolution of the material, making it easily treatable for the subsequent steps of selective recovery of the contained metals.

Although the use of organic acids on metal matrices is known, they are still little used for industrial purposes and, above all, they utilize pure commercial reagents, contributing to the consumption of natural resources.

In its preferred embodiments, the controlled fermentation process of the invention allows to obtain, at the end of the fermentation, a mixture with the desired requirements:

- ◦ Mixture A: pH = 6, maximum concentration of lactate obtained by almost total conversion of carbohydrates (lactose);

- ◦ Mixture B: lactic acid/lactate buffer pH = 3.8-4.0, high concentration of lactic acid/lactate obtained by almost total conversion of carbohydrates (lactose).

These results would not be achievable satisfactorily and in a cost-effective manner using the methods of the known art which require extensive use of reagents for continuous pH control and/or the addition of selected bacterial inocula.

The fermentation process as described in the present invention allows:

- optimization of the treatment steps, minimizing the reagents used, the number and duration of the process steps, mild operating conditions and low-cost plant solutions;

- maximum enhancement of all the components contained in the dairy waste (from lactobacilli to the nutrients contained in the waste);

- obtaining valuable secondary raw materials that can be directly used in an industrial field of great interest and growth such as that of critical metals recovery, thus creating an economically sustainable recovery chain (see Figure 1).

Further advantages of the invention:

- inexpensive fermentation processes applied to whey/scald without resorting to, or limiting as much as possible, the use of selected reagents and/or bacterial inocula in order to ensure high productivity and conversion efficiencies,

- the raw product can be sent directly to the fermentation step for use as a commercial grade leaching blend based on lactic acid/lactates. There are no known cases wherein the fermented product as such is directly used in hydro- or solvo-metallurgical processes;

- it is easy to identify the appropriate control parameters that allow in a single step and without the addition of external inoculum, an efficient and exclusive conversion of carbohydrates to lactic acid and a final pH compatible with leaching (3.8-6.5);

- low environmental impact and production of recovery mixtures suitable for the complete enhancement of waste for the treatment of hard metal and WEEE;

- potentially low-impact hydro-metallurgical processes using "green" reagents for leaching, which represent a lower operating cost and produce waste that is easier to inertize (waste water) and are optimized in terms of logistics, efficiency and costs, to the point of constitute a real industrial alternative to the methods in use.

- safe product, usable with bare hands, which can also be used as a home product for cleaning metals, above all because it can be marketed in various forms including the liquid or powder formulation to be reconstituted, for example to be dissolved in water or other liquids; another formulation, the cream, is particularly advantageous as it can be spread on the object to be cleaned, washing it off with water after cleaning. The cream form can be obtained as an intermediate product of drying the liquid form.

The invention will be further described by the following Examples which are to be considered illustrative and not limiting of the scope of the invention.

EXAMPLES

EXAMPLE 1 - FERMENTATION PROCESS FOR ACID LEACHING (MIXTURE B)

In the Example described, the anaerobic fermentation was carried out on 1.8 L of sheep's milk whey, in the dark (DF), in "batch" mode in a closed reactor with a volume equal to 2L, at a temperature of 39±1°C, under constant stirring (120 rpm), at atmospheric pressure after conditioning the headspace with nitrogen to remove air. Whey was characterized and used without pretreatment or addition of any external inoculum. Table 1 summarizes the main chemical-phisical characteristics of the sheep's milk whey used, as well as the characteristics of the fermented whey (FW) and the fermented whey after purification (PFW) for the purpose of obtaining **mixture B.**

**Table 1. Chemical-phisical characterization of the sheep whey used**

| **Parameter** | **Metering unit** | WHEY | FERMENTED WHEY **(FW)** | PURIFIED FERMENTED WHEY **(PFW)** |
|---|---|---|---|---|
| pH | - | 6.6 | 4.0 | n.d. |
| Total carbohydrates * | $g\ L^{-1}$ | 38.1 | 3.0 | 3.0 |
| Lactic acid | $g\ L^{-1}$ | 3.1 | 38.5 | 38.5 |
| Soluble Proteins ** | $g\ L^{-1}$ | 12.4 | 8 | 1.9 |
| * expressed as lactose<br>** expressed as bovine serum albumin (BSA) | | | | |

The pH was kept at the value of 6 with an automatic system capable of dosing a strong inorganic base (5M NaOH aqueous solution). The whey used was thawed at room temperature for about 8 hours. The parameter $q_k$ = $[HLat+lactates]_k/[carbo]_k$ was monitored by carrying out periodic withdrawals (every two hours for the first 12 hours of fermentation and every hour for the following 12 hours) and determining [HLat+lactates] by spectrophotometric method (Borshchevskaya L. N., Gordeeva T. L., Kalinina A.N., Sineokii S.P. 2016. "Spectrophotometric Determination of Lactic Acid" State Research Institute of Genetics and Selection of Industrial Microorganisms, Pervyi Dorozhnyi proezd 1, Moscow, 117545 Russia) and/or HPLC. When $q_k$ = $[HLat+lactates]_k/[carbo]_k$ reached a value of 4.8, the automatic pH control was interrupted and the natural end of the fermentative reaction was awaited, determined by the depletion of the carbohydrates initially present and the simultaneous achievement of a pH value ≤ 4 (Table 1).

The fermented whey (FW) thus obtained was purified through an initial sedimentation for 24 hours at a

temperature of 4°C which allowed the removal of a first part consisting essentially of microbial biomass, subsequently the supernatant was mixed with ethanol in a volumetric ratio equal to 3:5=EtOH:FW, stirred for 1h and settled for 24h. At the end of the 24h, the supernatant of the EtOH:FW mixture was centrifuged and further treated under vacuum using a rotatory evaporator for the recovery of ethanol.

EXAMPLE 2- FERMENTATION PROCESS FOR A LEACHING AGENT at pH = 6 (MIXTURE A)

In the Example described, the anaerobic fermentation was carried out on 1.8 L of sheep whey, in the dark (DF), in "batch" mode in a closed reactor with a volume equal to 2 L, at a temperature of 39$\pm$1°C, under constant stirring (120 rpm), at atmospheric pressure, after conditioning the headspace with nitrogen to remove air. Ovine whey was characterized and used without pretreatment or addition of any external inoculum. In this case, the fermented whey was treated under conditions of controlled pH = 6 until the completion of the conversion of carbohydrates to lactates (i.e., 98% of the carbohydrates present had been converted to lactate) and then subjected to a first decantation/separation of the insoluble organic matter, then drying in an oven at 50-60°C (see Fig. 4a). Ethanol washing of the dried fermented whey allowed the lactate salts to be extracted from the whole solid mass to solution, leaving the organic matter insoluble in ethanol (see Fig.4b).

The main advantages of this approach are linked to the possibility of obtaining, in a single step, an organic solution of purified lactate at the desired concentration.

EXAMPLE 3 - Selective leaching of base (E°<0) and noble (Ag, Cu; E°>0) metals from shredded WEEE.

The test sample used in this Example consists of the fine fraction (average particle size: 0.4 mm) of non-ferrous metals from electronic boards (PCB) small electronic equipment, e.g. mobile phones, notebooks, etc., chopped and reduced to powder as supplied by a company that treats WEEE, by applying a mechanical shredding and separation process. The metal content of the shredded WEEE sample was determined by ICP-AES analysis (inductively coupled plasma source atomic emission spectroscopy; Perkin Elmer Optima DV 7000) after treatment in a Mileston Ethos 1 microwave digester (Serpe et al., 2015). The average metallic composition of the sample is: (%, w/w) Cu 79; Sn 10; Pb 7; Zn 2; Ni 0.5; Ag 0.06; Au 0.01; Others (Al, Cr, Mn, Fe, Co) 1.43.

In order to evaluate the behavior of the different metals in PFW (Mixture B, pH $\leq$4, [HLat] = [Lat] = 0.25M) under varying conditions, the tests were carried out on 1 g of sample as detailed in the tables 2 and 3, respectively for tests 1 and 2:

**Table 2. Operating parameters of test 1:**

| Step | T | Volume PFW [mL] | Other reagents | T [h] | Final solution |
|---|---|---|---|---|---|
| 1 | Ambient | 200 | - | 8 | Sol. 1 |
| 2 | Reflux | 200 | - | 24 | Sol. 2 |
| 3 | Reflux | 200 | 1.5mL $H_2O_2$ | 48 | Sol. 3 |
| 4 | Reflux | 50 | 0.5mL $H_2O_2$ | 24 | Sol. 4 |

**Table 3. Operating parameters of test 2**

| Step | T | Volume PFW [mL] | Other reagents | T [h] | Final solution |
|---|---|---|---|---|---|
| 1 | Ambient | 200 | - | 8 | Sol. 1 |
| 2 | Reflux | 200 | - | 24 | Sol. 2 |
| 3 | Ambient | 200 | 1.5mL $H_2O_2$ | 48 | Sol. 3 |
| 4 | Ambient | 50 | 0.5mL $H_2O_2$ | 24 | Sol. 4 |

Magnetic stirring was used in all tests.

The charts (a) shown in Figure 4 show the leaching profiles of the metals, by mass (mg) of metal dissolved over time determined by ICP-AES analysis. The profiles relating to the 2nd, 3rd and 4th leaching steps were constructed considering the cumulative masses of each metal relating to the sum of what was found in Solutions 1, 2, 3 and 4 of the specific test. The charts (b) show the % distribution of the dissolved metal between the 4 solutions (1, 2, 3 and 4) while the charts in Figure 5 show the % metal composition.

As can be deduced from the data, the PFW is able to leach significant quantities of Fe, Zn, Pb, Al and Sn already after 8h at $T_{amb}$, with a further increase in the following 8h at the reflux temperature which strongly improves the leaching efficiency of Ni. The reaction, under the indicated conditions, is selective with respect to the mentioned metals, i.e. those with the lowest reduction potential. Furthermore, the addition of $H_2O_2$ also allows efficient leaching of Cu and Ni which have higher potential, reaching leached fractions highly enriched in the various species: in solutions 1-2 about 75% of Fe, 60% of Zn, 50% of Pb, 80% of Al, 40% of Sn and 40% of Ni are concentrated; in solutions 3-4, on the other hand, we find over 90% of copper and a further 40% of Ni. This demonstrates the significant leaching capacity of Mixture B and its selectivity as the operating conditions vary. Similar results were obtained on a sample of shredded RAM cards without resorting to physical separation pre-treatments of the non-metallic and magnetic metallic fraction. Table 4 shows a comparison between the leaching yields of the different metals contained in the shredded cards by treating 1 g of sample with similar quantities of commercial HLat solution and PFW (L/S = 50) at pH 4 both at room temperature for 48 hours and at 100°C in a microwave oven for 12 hours in closed vessels. As can be deduced from the comparison of the data, the leaching yields of the metals in PFW are comparable if not even higher than those obtained from the model solution. In particular, as also shown in Figure 7, Fe and Ni prove to be particularly reactive in PFW. It should also be noted that the use of PFW optimizes the separation by precipitation of Pb thanks to the presence in the mixture of significant quantities of chlorides.

**Table 4** - **Metal leaching yields from 1g of shredded WEEE with commercial HLat and PFW (pH = 4, [HLat] = [$Lat^-$] = 0.25M, L/S = 50) for 48 h at $T_{amb}$ (A); for 24 hours at 100°C in a microwave oven.**

| Metal | A: Leaching yields (%) | | B: Leaching yields (%) | |
|---|---|---|---|---|
| | Commercial HLat | PFW | Commercial HLat | PFW |
| Fe | 10 | 73 | 33 | 60 |
| Cu | 15 | 15 | 1 | 1 |
| Pb | 97 | 98 | 100 | 65 |
| Al | 7 | 17 | 4 | 12 |
| Sn | 41 | 61 | 50 | 58 |
| Ni | 13 | 72 | 39 | 60 |

EXAMPLES 4A and 4B - Selective leaching of Co from waste WC-Co powders from the production of "hard metals".

The experiments object of the present Examples were carried out on a WC-(20%)Co powder obtained as a by-product of the forming processes of the hard metal pieces during the steps of the production process.

Ex. 4A: Leaching was performed on 5.18 g of sample using a fine bubble aeration system and mechanical stirring system on the solution for 4 hours at room temperature, using 0.400 L of PFW (mixture B, pH ≤4) and concentration 0.35 M, according to a molar ratio acid:Co 1:8. Under these conditions, the 4-hour leaching resulted in the dissolution of 50% Co on the real material obtaining a recycled WC-Co powder with 9.6% Co. This result highlights the direct applicability of Leaching Mixture B on powders based on of WC-Co for the selective dissolution of Co and suggests appropriately calibrating the leaching conditions according to the desired Co removal rate: e.g. adopt longer leaching times and/or solution concentration processes if aiming to obtain higher dissolution yields and/or more favorable L/S ratios for industrial purposes.

Ex. 4B: The leaching experiment was carried out on 32.5 g of the sample powder described above, with 0.440 L of a [$Lat^-$]=1M solution obtained by drying the FW (Mixture A, pH = 6) and extracting the lactate salts component with a suitable quantity of EtOH. The powder was reacted under magnetic stirring for 24 hours in the presence of $I_2$ as oxidant in a Co:$Lat^-$:$I_2$ molar ratio equal to 1:4:1. The chemical characterization of the leached matter and the solid residue showed a selective leaching yield of Co equal to 60% (recovered solid residue: WC-(8%)Co) which demonstrates the effectiveness of the solvo-metallurgical complexation/oxidation process obtained in ethanol starting from FW in the presence of iodine. Also in this case, a different combination of experimental conditions (leaching time, concentration, temperature) can affect the % composition of the fractions obtained.

EXAMPLE 5. Dissolution of base metals and "rare earths" from alloys or oxides.

The experiments object of the present Example were carried out on a powder obtained by thermal treatment and shredding of $Nd_2Fe_{14}B$ permanent magnets from hard disks (Fig. 8A). NdFeB alloy magnets are among the strongest permanent magnets on the market today. The general composition of the alloy is $Nd_2Fe_{14}B$ but other metals are often present, including Dy and Ni, in small quantities, whose percentage often varies according to the type of application. Ni is

used as a coating to avoid oxidation of the alloy. To carry out the tests, the extracted magnets were subjected to partial demagnetization by heating them at 200°C for one hour (Fig. 8B). This treatment allowed the easy manual removal of the nickel coating from the magnet (Fig. 8C) thus favoring the oxidation of the alloy components in order to allow the crushing, characterization and grinding of the piece.

The XRD characterization on the fragments of the heat-treated magnet stripped of the nickel coating show that alongside the typical peaks of the neodymium-iron-boron alloy, $Nd_2Fe_{14}B$, there are peaks that can be attributed to praseodymium III oxide, $Pr_2O_3$ and of neodymium IV oxide, $NdO_2$.

Aliquots of 15 mg each of this material reduced to powder by mechanical shredding were treated at room temperature and under constant stirring:

- with a commercial lactic acid solution brought to pH = 4 by adding NaOH (aq);
- with Mixture B (pH ≤4) from the above example

obtaining in both cases the complete dissolution of all the solid material treated, both present in the form of metal alloy and in the form of oxides, already within 2-3 hours, obtaining a light yellow solution which can then be treated for selective extraction of different metals.

These results are of absolute relevance as they show how the mixture of the invention, safe and eco-compatible, is effective in mobilizing species starting from a highly resistant material and initiating subsequent selective recovery and recycling steps, and represents a valid alternative to strong acids and oxidants, often dangerous.

Tests on whole fragments of magnet led to the same result, requiring, for the same mass of the sample, slightly longer leaching times (about doubled).

## Claims

**1.** A process for the production of a leaching composition from raw dairy product waste comprising the following steps:

- Carrying out a bacterial fermentation in liquid phase in the absence of light and under anaerobic conditions on the raw waste, controlling the pH by adding a base so that it is kept in the range of about 4.5-6.5, preferably about 5.0-6.0; particularly preferred is the range about 5.5-6.0;
- Controlling the fermentation by taking samples to determine the molar concentration of lactic acid and lactates as well as carbohydrates in the fermenting mix both at the beginning and during the fermentation process to calculate the monitoring parameter

$$q_k = [HLat+lactates]_k/[carbo]_k$$

- Wherein:

  - $[HLat+lactates]_k$ = molar concentration of lactic acid+lactates upon $k^{th}$ sampling measured with at least 2 significant digits,
  - $[carbo]_k$ = molar concentration of carbohydrates in the liquid waste upon $k^{th}$ sampling, measured with at least 2 significant digits,
  - k = positive integer greater than 0;

- Continuing the fermentation until the parameter $q_k = [HLat+lactates]_k/[carbo]_k$ is in the range between about 4 and about 5, then stop adding base and letting the reaction continue spontaneously until the end of the fermentation;
- Recovering the leaching composition by separation from the reaction bacterial biomass;
- Optionally purifying the obtained leaching composition to eliminate proteins and impurities;
- Optionally operating a drying or dehydration step to obtain a dried or dehydrated final product.

**2.** The process according to the preceding claim wherein the end of the fermentation is identified by the achievement of about 95% or greater of the carbohydrates converted into lactates.

**3.** The process according to any one of claims 1-2 wherein k is a discrete quantity associated with the number of subsequent withdrawals distant over time of at least 30 minutes.

4. The process according to any one of claims 1-3 wherein the raw waste is selected from milk whey and scald.

5. The process according to any one of claims 1-3 wherein the raw waste derives from sheep, goat, cow, buffalo or other animal milk from which milk is obtained to produce dairy products.

6. The process according to any one of claims 1-5 wherein the raw waste has been previously refrigerated, frozen and thawed, dried or dehydrated or freeze-dried and subsequently reconstituted.

7. The process according to claim 6 wherein the dried or dehydrated or freeze-dried crude waste is reconstituted in water, or is mixed with thawed whey or crude whey, or is added to the residual biomass deriving from the process according to claims 1-2.

8. The process according to any one of claims 1-7 wherein the leaching composition obtained is used as leaching agent as such after separation by decantation of the solid biomass, or after treatment with alcohols, for example ethanol to purify the composition from the protein part.

9. Leaching composition obtained according to any one of claims 1-8 **characterized by** a pH ranging from about 6 to 3.8 and wherein the lactate/lactic acid ratio varies from about all lactate to about 1:1, respectively.

10. The leaching composition of claim 9 having a pH about 6 and negligible or no lactic acid content compared to the lactate content.

11. The leaching composition obtained according to any one of claims 2-8 **characterized by** a pH $\leq 4$ and wherein the lactate/lactic acid ratio is about 1:1.

12. The leaching composition according to any one of claims 9-11 which is in liquid form, fluid form, cream, dried form or dried and reconstituted with water or solvents after drying or dehydration or lyophilization form.

13. Use of the composition according to any one of claims 9-12 as a leaching agent for the recovery treatment of metals from scrap metal materials, in particular in the fields of hydro-metallurgy and solvo-metallurgy.

14. The use of the composition according to any one of the claims 9-12 as a leaching agent for the treatment of scraps of the hard metal chain and their semi-finished products, in order to recover the critical metals including W and Co in the presence or absence of an added oxidant and wherein the selected oxidant is preferably $O_2$, $I_2$, $H_2O_2$.

15. The use of the composition according to any one of claims 9-12 as a leaching agent for the treatment of waste electrical and electronic equipment, including permanent magnets, and semi-finished products thereof, in order to recover critical metals including base metals, rare earths, noble metals; the treatment with the leaching agent being carried out in the absence or in the presence of an added oxidant and wherein the oxidant is preferably selected from among $O_2$, $I_2$, $H_2O_2$.

**Patentansprüche**

1. Verfahren zur Herstellung einer Auslaugungszusammensetzung aus rohen Molkereiabfällen, umfassend die folgenden Schritte :

   - Durchführung einer bakteriellen Fermentation der Rohabfälle in flüssiger Phase unter Lichtausschluss und anaeroben Bedingungen, wobei der pH-Wert durch Zugabe einer Base im Bereich von etwa 4,5-6,5, vorzugsweise etwa 5,0-6,0, gehalten wird; besonders bevorzugt ist der Bereich von etwa 5,5-6,0;
   - Die Fermentation wird durch Probenahme gesteuert, um die molare Konzentration von Milchsäure und Laktaten sowie Kohlenhydraten in der Fermentationsmischung zu Beginn und während des Fermentationsprozesses zu bestimmen und den Überwachungsparameter zu berechnen:

$$q_k = [HLat+Laktate]_k/[Kohlenhydrate]_k$$

   wobei :

- $[HLat+Laktate]_k$ = molare Konzentration von Milchsäure und Laktaten bei der k-ten Probenahme, mit mindestens 2 signifikanten Ziffern),
- $[Kohlenhydrate]_k$ = molare Konzentration der Kohlenhydrate im flüssigen Abfall bei der k-ten Probenahme, mit mindestens 2 signifikante Ziffern),
- k = positive ganze Zahl größer als 0;
- Die Fermentation wird fortgesetzt, bis der Parameter $q_k = [HLat+Laktate]_k/[Kohlenhydrate]_k$ im Bereich zwischen etwa 4 und etwa 5 liegt. Anschließend wird die Basenzugabe gestoppt und die Reaktion spontan bis zum Ende der Fermentation fortgesetzt;
- Die Zusammensetzung des Auslaugungsprodukts wird durch Abtrennung der bakteriellen Biomasse aus der Reaktion gewonnen;
- Optional: Reinigung der erhaltenen Auslaugungszusammensetzung zur Entfernung von Proteinen und Verunreinigungen;
- Optional: Durchführung eines Trocknungs- oder Dehydratisierungsschritts zur Gewinnung eines getrockneten oder dehydratisierten Endprodukts.

**2.** Verfahren nach dem vorhergehenden Anspruch, wobei das Ende der Fermentation durch die Umwandlung von etwa 95 % oder mehr der Kohlenhydrate in Laktate gekennzeichnet ist.

**3.** Verfahren nach einem der Ansprüche 1 bis 2, wobei k eine diskrete Größe ist, die mit der Anzahl aufeinanderfolgender Probenahmen im Abstand von mindestens 30 Minuten verbunden ist.

**4.** Verfahren nach einem der Ansprüche 1 bis 3, wobei der Rohabfall aus Molke und Brühwasser ausgewählt ist.

**5.** Verfahren nach einem der Ansprüche 1 bis 3, wobei der Rohabfall aus Schaf-, Ziegen-, Kuh-, Büffel- oder anderer Tiermilch stammt, aus der Milch zur Herstellung von Milchprodukten gewonnen wird.

**6.** Verfahren nach einem der Ansprüche 1 bis 5, wobei der Rohabfall zuvor gekühlt, gefroren und aufgetaut, getrocknet, dehydriert oder gefriergetrocknet und anschließend rekonstituiert wurde.

**7.** Verfahren nach Anspruch 6, wobei der getrocknete, dehydrierte oder gefriergetrocknete Rohabfall in Wasser rekonstituiert, mit aufgetauter Molke oder Rohmolke vermischt oder der aus dem Verfahren nach den Ansprüchen 1 bis 2 stammenden Restbiomasse zugesetzt wird.

**8.** Verfahren nach einem der Ansprüche 1 bis 7, wobei die erhaltene Auslaugungszusammensetzung nach Abtrennung der festen Biomasse durch Dekantieren oder nach Behandlung mit Alkoholen, beispielsweise Ethanol, zur Reinigung der Zusammensetzung vom Proteinanteil als Auslaugungsmittel verwendet wird.

**9.** Auslaugungszusammensetzung nach einem der Ansprüche 1 bis 8, **gekennzeichnet durch** einen pH-Wert von etwa 6 bis 3,8 und ein Lactat/Milchsäure-Verhältnis von nahezu reinem Lactat bis etwa 1:1.

**10.** Auslaugungszusammensetzung nach Anspruch 9 mit einem pH-Wert von etwa 6 und einem im Vergleich zum Lactatgehalt vernachlässigbaren oder nicht vorhandenen Milchsäuregehalt.

**11.** Auslaugungszusammensetzung nach einem der Ansprüche 2 bis 8, **gekennzeichnet durch** einen pH $\leq$ 4 und ein Lactat/Milchsäure-Verhältnis von etwa 1:1.

**12.** Auslaugungszusammensetzung nach einem der Ansprüche 9 bis 11 in flüssiger, fluider, getrockneter oder getrockneter und nach Trocknung, Dehydratisierung oder Lyophilisierung mit Wasser oder Lösungsmitteln rekonstituierter Form.

**13.** Verwendung der Zusammensetzung nach einem der Ansprüche 9 bis 12 als Laugungsmittel zur Rückgewinnung von Metallen aus Metallschrott, insbesondere im Bereich der Hydrometallurgie und Solvometallurgie.

**14.** Verwendung der Zusammensetzung nach einem der Ansprüche 9 bis 12 als Laugungsmittel zur Behandlung von Schrott der Hartmetallkette und deren Halbzeugen zur Rückgewinnung der kritischen Metalle, einschließlich W und Co, mit oder ohne Zusatz eines Oxidationsmittels, wobei das gewählte Oxidationsmittel vorzugsweise $O_2$, $I_2$ oder $H_2O_2$ ist.

**15.** Verwendung der Zusammensetzung nach einem der Ansprüche 9 bis 12 als Laugungsmittel zur Behandlung von Elektro- und Elektronikschrottgeräten, einschließlich Permanentmagneten, und deren Halbzeugen zur Rückgewinnung der kritischen Metalle, einschließlich unedler Metalle, Seltenerdmetalle und Edelmetalle; wobei die Behandlung mit dem Auslaugungsmittel wird in Abwesenheit oder in Gegenwart eines zugesetzten Oxidationsmittels durchgeführt, wobei das Oxidationsmittel vorzugsweise aus $O_2$, $I_2$ oder $H_2O_2$ ausgewählt ist.

## Revendications

**1.** Procédé de production d'un mélange de lixiviation à partir de déchets bruts de produits laitiers comprenant les étapes suivantes :

- Effectuer une fermentation bactérienne en phase liquide, à l'abri de la lumière et en conditions anaérobies, sur les déchets bruts, avec contrôle du pH par ajout d'une base afin de le maintenir dans la plage d'environ 4,5 à 6,5, de préférence entre 5,0 et 6,0 ; la plage de 5,5 à 6,0 étant particulièrement préférée.
- Contrôler la fermentation en prélevant des échantillons pour déterminer la concentration molaire d'acide lactique, de lactates et de glucides dans le mélange de fermentation, au début et en cours du processus de fermentation, afin de calculer le paramètre de suivi :

$$q_k = [HLat+lactates]_k/[carbo]_k$$

où :

  - $[HLat+lactates]_k$ = concentration molaire d'acide lactique + lactates au k-ième prélèvement, mesurée avec au moins 2 chiffres significatifs ;
  - $[carbo]_k$ = concentration molaire de glucides dans le déchet liquide au k-ième prélèvement, mesurée avec au moins 2 chiffres significatifs ;
  - k = entier positif supérieur à 0 ;

- Poursuivre la fermentation jusqu'à ce que le paramètre $q_k = [HLat+lactates]_k/[carbo]_k$ se trouve dans la plage entre 4 et 5 environ, puis arrêter l'ajout de base et laisser la réaction se poursuivre spontanément jusqu'à la fin de la fermentation ;
- Récupérer le composé de lixiviation par séparation de la biomasse bactérienne de la réaction ;
- Purification optionnelle de la composition de lixiviation obtenue afin d'éliminer les protéines et les impuretés ;
- Opérer un séchage ou déshydratation optionnels pour obtenir un produit final séché ou déshydraté.

**2.** Procédé selon la revendication précédente, dans lequel la fin de la fermentation est identifiée par l'obtention d'environ 95 % ou plus des glucides convertis en lactates.

**3.** Procédé selon l'une quelconque des revendications 1 à 2, dans lequel k est une grandeur discrète associée au nombre d'échantillonnages successifs espacés d'au moins 30 minutes.

**4.** Procédé selon l'une quelconque des revendications 1 à 3, dans lequel les déchets bruts sont sélectionnés parmi le lactosérum et les résidus de cuisson.

**5.** Procédé selon l'une quelconque des revendications 1 à 3, dans lequel les déchets bruts proviennent du lait de brebis, de chèvre, de vache, de bufflonne ou d'autres animaux dont le lait est utilisé pour la production de produits laitiers.

**6.** Procédé selon l'une quelconque des revendications 1 à 5, dans lequel les déchets bruts ont été préalablement réfrigérés, congelés et décongelés, séchés, déshydratés ou lyophilisés, puis reconstitués.

**7.** Procédé selon la revendication 6, dans lequel les déchets bruts séchés, déshydratés ou lyophilisés sont reconstitués dans l'eau, ou mélangés à du lactosérum décongelé ou à du lactosérum brut, ou ajoutés à la biomasse résiduelle issue du procédé selon les revendications 1 à 2.

**8.** Procédé selon l'une quelconque des revendications 1 à 7, dans lequel la composition de lixiviation obtenue est utilisée comme agent de lixiviation telle quelle après séparation par décantation de la biomasse solide, ou après traitement avec des alcools, par exemple de l'éthanol, pour purifier la composition de sa fraction protéique.

**9.** Composition de lixiviation obtenue selon l'une quelconque des revendications 1 à 8, **caractérisée par** un pH compris entre environ 6 et 3,8 et dans laquelle le rapport lactate/acide lactique varie de la quasi-totalité du lactate à environ 1:1.

**10.** Composition de lixiviation selon la revendication 9, présentant un pH d'environ 6 et une teneur en acide lactique négligeable ou nulle par rapport à la teneur en lactate.

**11.** Composition de lixiviation obtenue selon l'une quelconque des revendications 2 à 8, **caractérisée par** un pH ≤ 4 et dans laquelle le rapport lactate/acide lactique est d'environ 1:1.

**12.** Composition de lixiviation selon l'une quelconque des revendications 9 à 11, se présentant sous forme liquide, fluide, crémeuse, lyophilisée ou lyophilisée puis reconstituée avec de l'eau ou des solvants après séchage, déshydratation ou lyophilisation.

**13.** Utilisation de la composition selon l'une quelconque des revendications 9 à 12 comme un agent de lixiviation pour le traitement de récupération des métaux contenus dans les déchets métalliques, notamment dans les domaines de l'hydrométallurgie et de la solvométallurgie.

**14.** Utilisation de la composition selon l'une quelconque des revendications 9 à 12 comme un agent de lixiviation pour le traitement des déchets de la chaîne de métaux durs et de leurs produits semi-finis, afin de récupérer les métaux critiques, notamment W et Co, en présence ou en absence d'un oxydant ajouté, l'oxydant choisi étant de préférence $O_2$, $I_2$ ou $H_2O_2$.

**15.** Utilisation de la composition selon l'une quelconque des revendications 9 à 12 comme un agent de lixiviation pour le traitement des déchets d'équipements électriques et électroniques, y compris les aimants permanents, et de leurs produits semi-finis, afin de récupérer les métaux critiques, notamment les métaux de base, les terres rares et les métaux précieux, le traitement avec l'agent de lixiviation étant effectué en l'absence ou en présence d'un oxydant ajouté et dans lequel l'oxydant est de préférence choisi parmi $O_2$, $I_2$, $H_2O_2$.

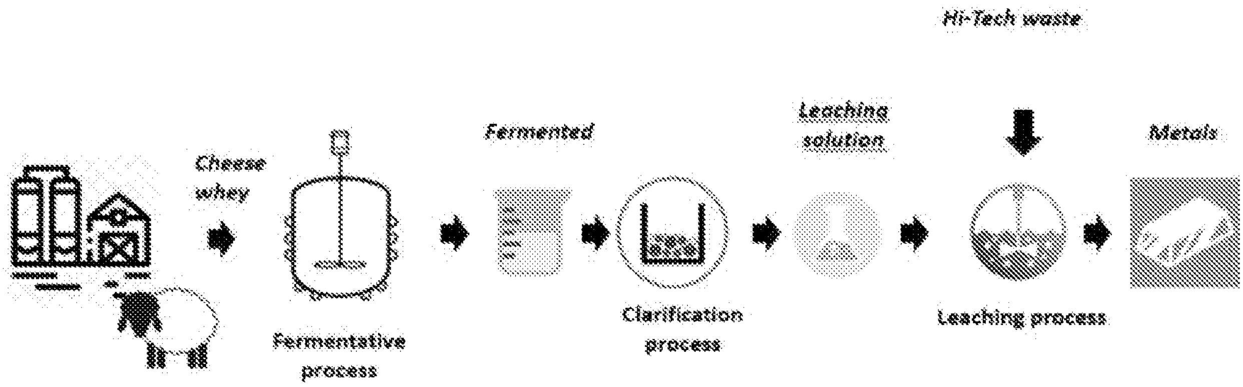
Hi-Tech waste
Cheese
whey
Fermented
Leaching
solution
Metals
Fermentative
process
Clarification
process
Leaching process

Fig. 1.

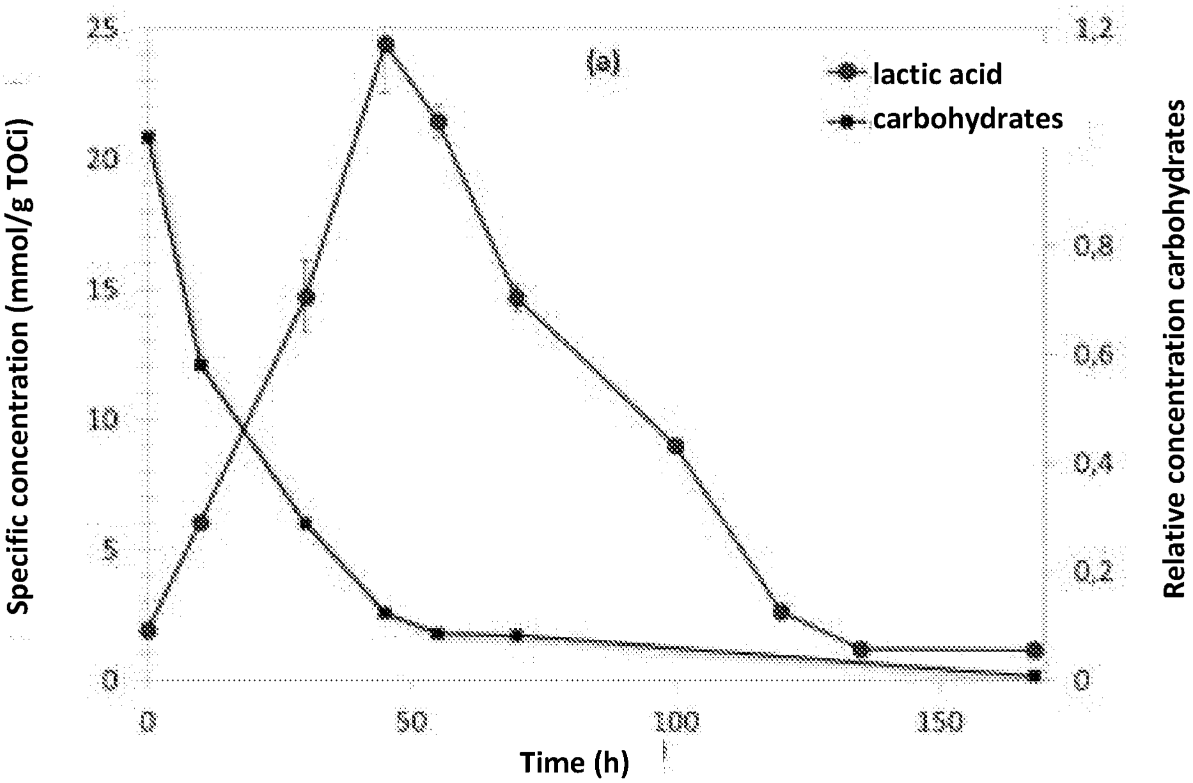
(a)
lactic acid
carbohydrates
Specific concentration (mmol/g TOCi)
Relative concentration carbohydrates
Time (h)
0
5
10
15
20
25
0
0,2
0,4
0,6
0,8
1,2
0
50
100
150

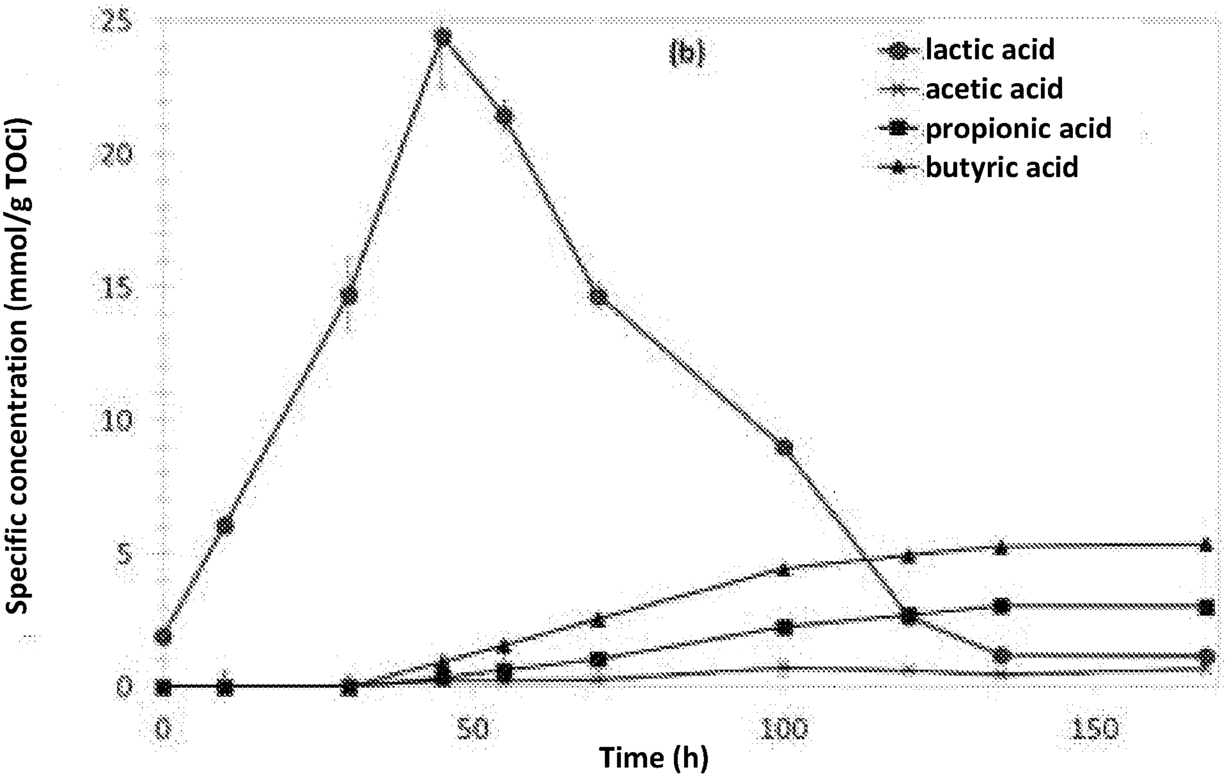
(b)
lactic acid
acetic acid
propionic acid
butyric acid
Specific concentration (mmol/g TOCi)
Time (h)
0
5
10
15
20
25
0
50
100
150

Fig. 2.

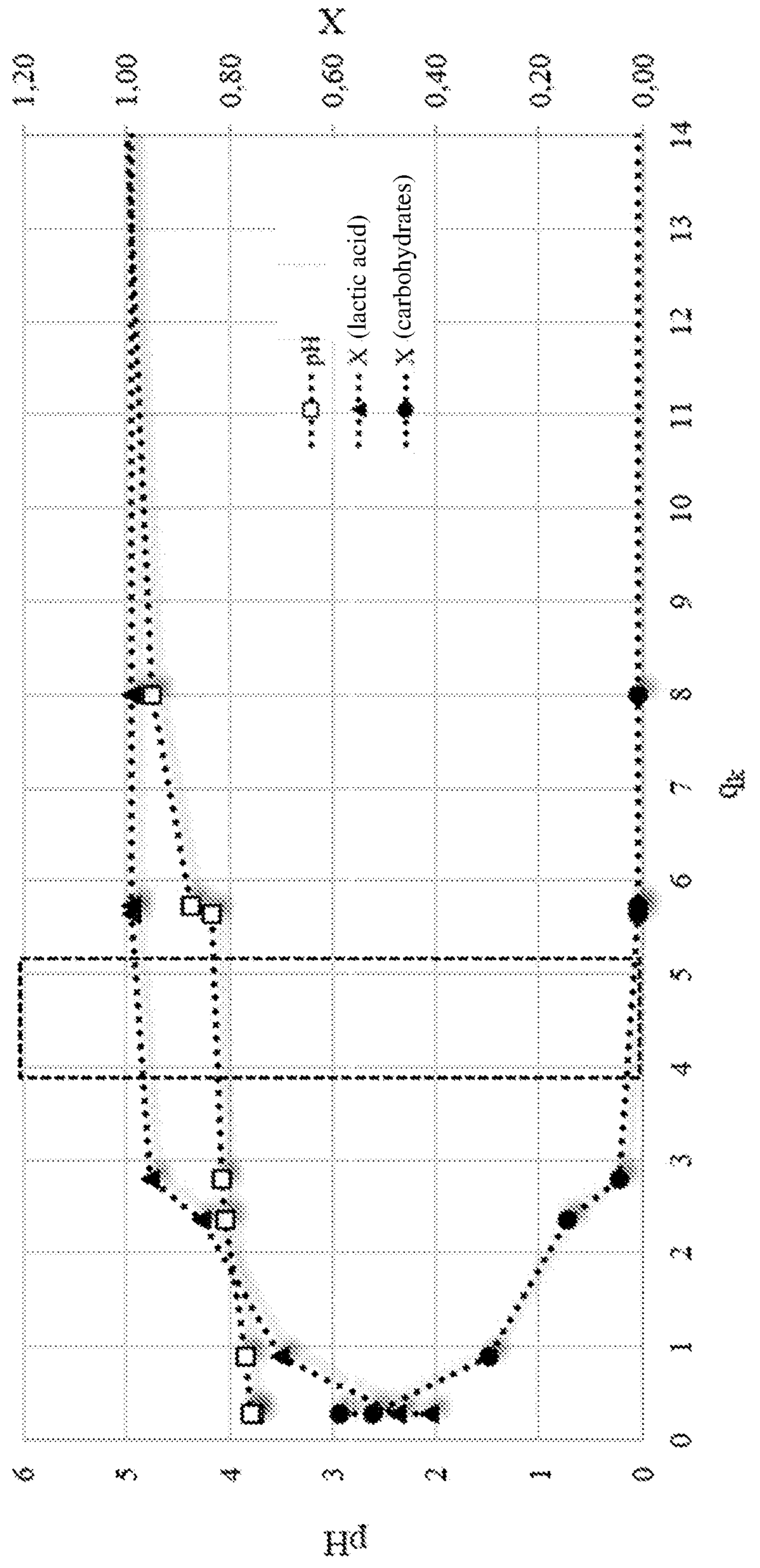
X
1,20
1,00
0,80
0,60
0,40
0,20
0,00
pH
X (lactic acid)
X (carbohydrates)
pH
0
1
2
3
4
5
6
0
1
2
3
4
5
6
7
8
9
10
11
12
13
14

Fig. 3.

(a) (b)

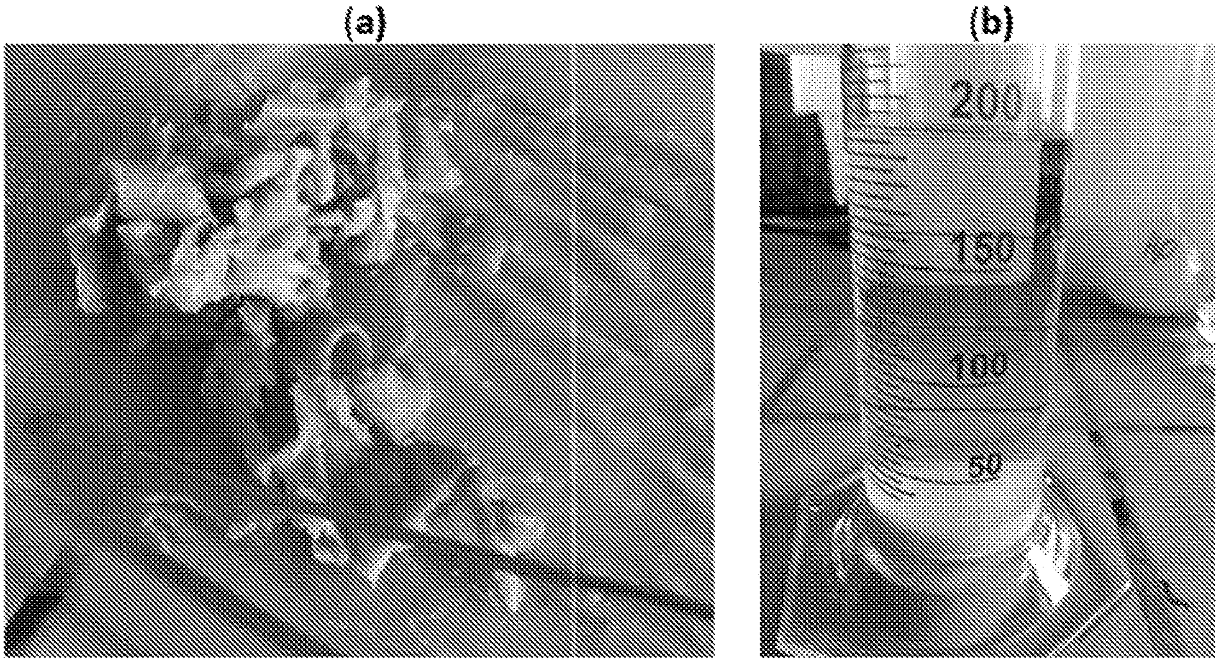


Fig. 4.

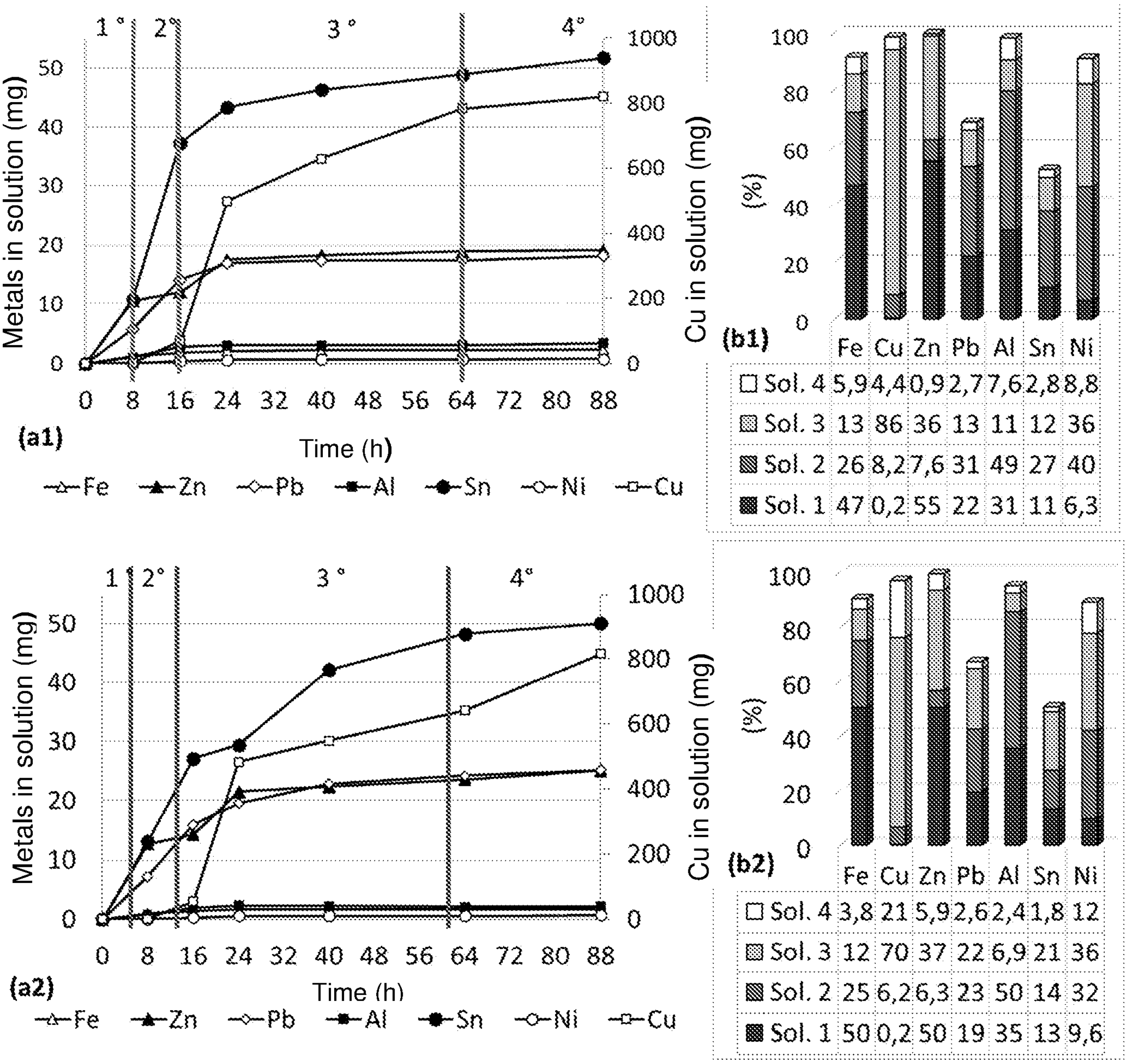
(a1)
1 °
2°
3 °
4°
Metals in solution (mg)
Cu in solution (mg)
Time (h)
Fe Zn Pb Al Sn Ni Cu
(b1)
(%)
Fe Cu Zn Pb Al Sn Ni
Sol. 4 5,9 4,4 0,9 2,7 7,6 2,8 8,8
Sol. 3 13 86 36 13 11 12 36
Sol. 2 26 8,2 7,6 31 49 27 40
Sol. 1 47 0,2 55 22 31 11 6,3
(a2)
1
2°
3 °
4°
Metals in solution (mg)
Cu in solution (mg)
Time (h)
Fe Zn Pb Al Sn Ni Cu
(b2)
(%)
Fe Cu Zn Pb Al Sn Ni
Sol. 4 3,8 21 5,9 2,6 2,4 1,8 12
Sol. 3 12 70 37 22 6,9 21 36
Sol. 2 25 6,2 6,3 23 50 14 32
Sol. 1 50 0,2 50 19 35 13 9,6

Fig. 5.

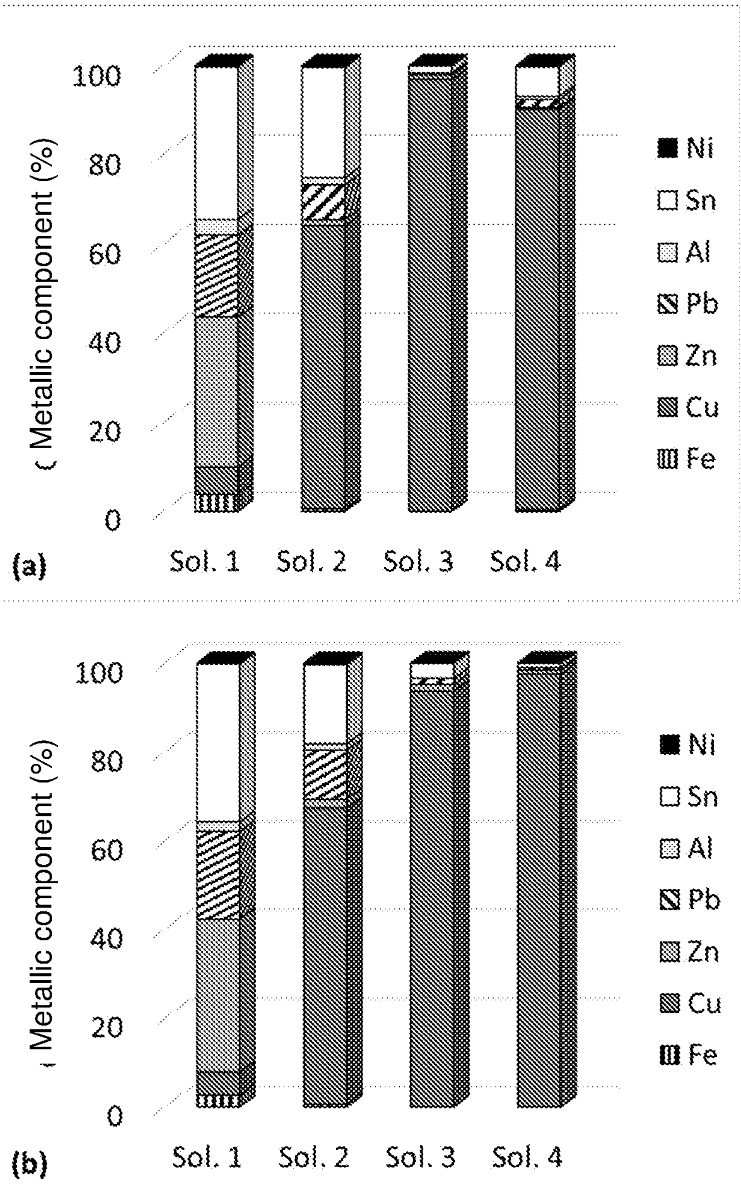


(a)

| | Composition % solutions | | | |
|---|---|---|---|---|
| | Sol. 1 | Sol. 2 | Sol. 3 | Sol. 4 |
| Fe | 4 | 1 | 0,05 | 0 |
| Cu | 6 | 64 | 97 | 90 |
| Zn | 34 | 1 | 1 | 0,40 |
| Pb | 18 | 8 | 0,46 | 2 |
| Al | 3 | 2 | 0,05 | 1 |
| Sn | 34 | 25 | 2 | 7 |
| Ni | 0,18 | 0,33 | 0,04 | 0,19 |

(b)

| | Composition % solutions | | | |
|---|---|---|---|---|
| | Sol. 1 | Sol. 2 | Sol. 3 | Sol. 4 |
| Fe | 3 | 1 | 0,04 | 0,04 |
| Cu | 5 | 67 | 94 | 98 |
| Zn | 34 | 2 | 1 | 1 |
| Pb | 20 | 11 | 1 | 1 |
| Al | 2 | 1 | 0,03 | 0,03 |
| Sn | 36 | 18 | 3 | 1 |
| Ni | 0,20 | 0,32 | 0,04 | 0,05 |

Fig. 6.

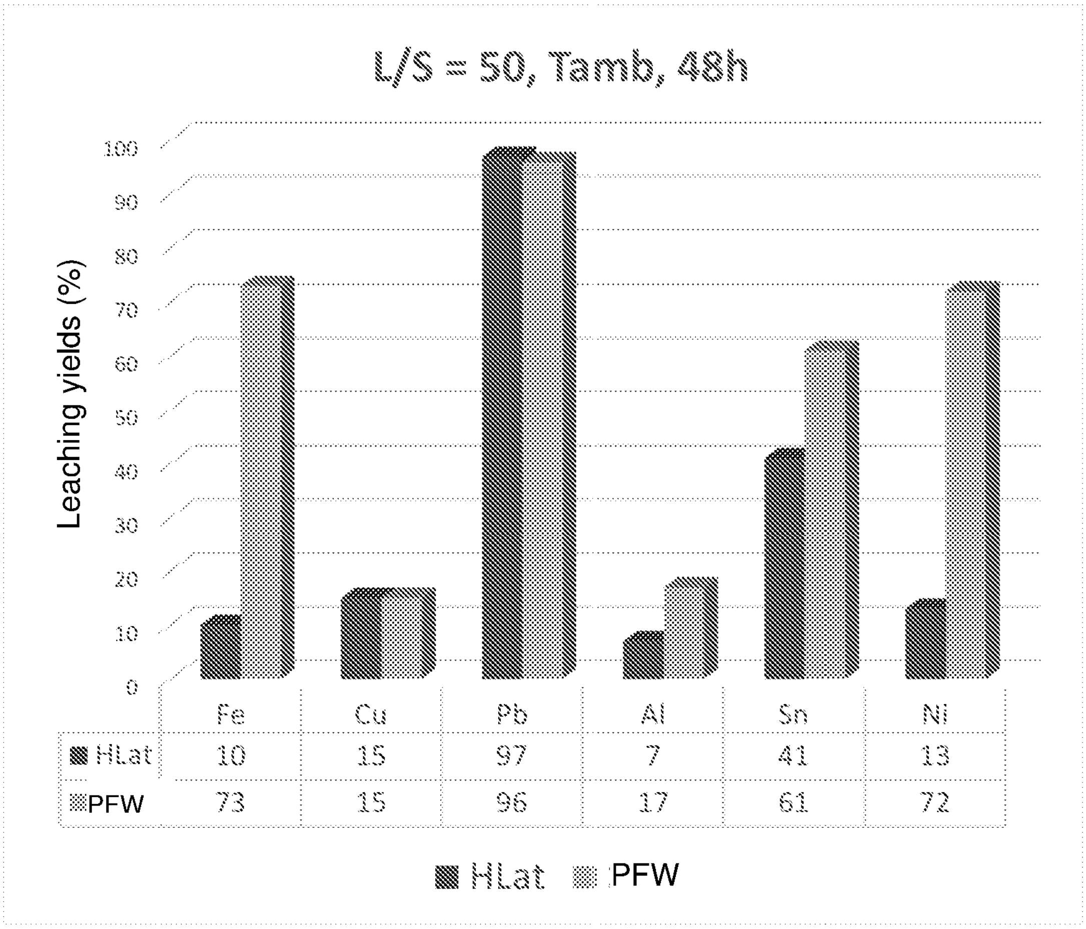
L/S = 50, Tamb, 48h
Leaching yields (%)
100
90
80
70
60
50
40
30
20
10
0
Fe Cu Pb Al Sn Ni
HLat 10 15 97 7 41 13
PFW 73 15 96 17 61 72
HLat PFW

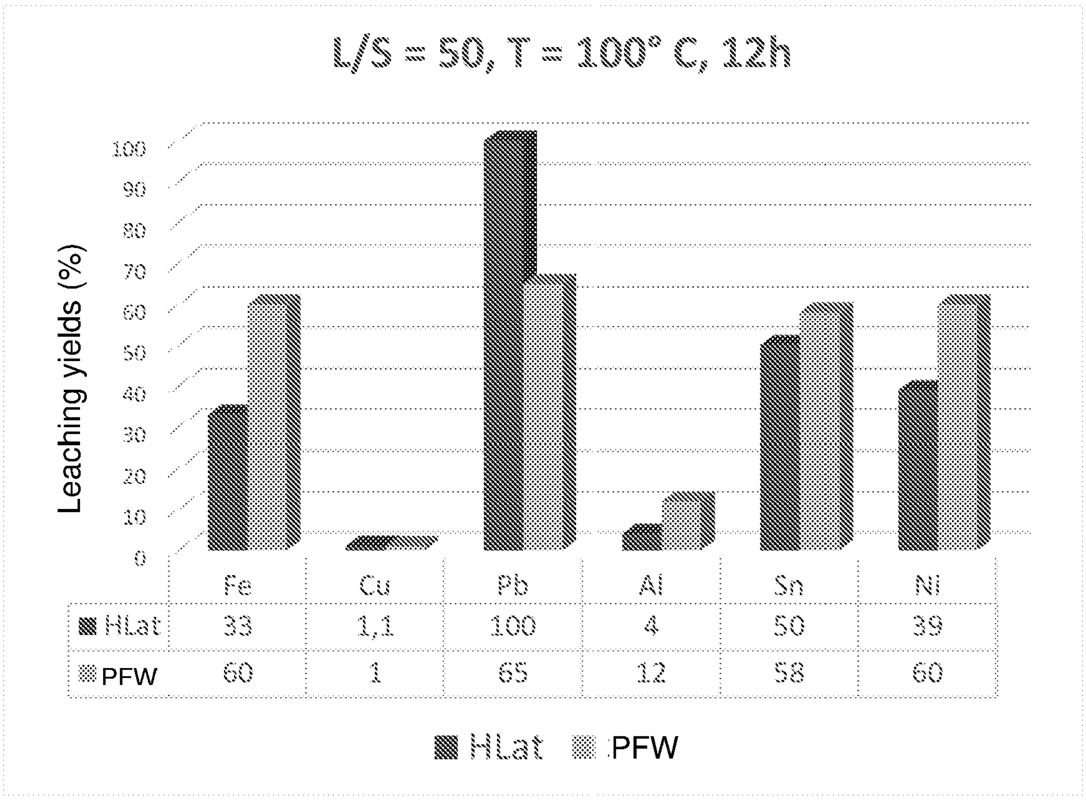
L/S = 50, T = 100° C, 12h
Leaching yields (%)
100
90
80
70
60
50
40
30
20
10
0
Fe Cu Pb Al Sn Ni
HLat 33 1,1 100 4 50 39
PFW 60 1 65 12 58 60
HLat PFW

Fig. 7.

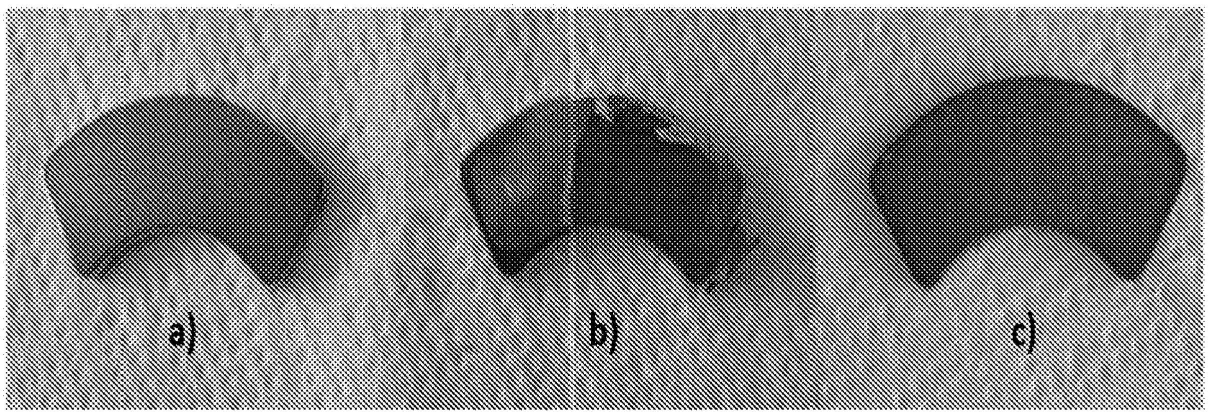


Fig. 8.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 3950977 A1
- NZ 287397 A

### Non-patent literature cited in the description


- **OUMAROU AMADOU, A.** ; **DE GAUDENZI, G.P.** ; **MARCHESELLI, G.** ; **CARA, S.** ; **PIREDDA, M.** ; **SPIGA, D.** ; **MATHARU, A.S** ; **DE GIOANNIS, G.** ; **SERPE, A. A**. New Easy Solvometallurgical Leaching Method for the Selective Co dissolution & Recovery from Hard Metals Waste (2021). *International Journal of Refractory Metals and Hard Materials*, 98
- **GHAFFAR et al.** *Journal of Radiation Research and Applied Sciences*, 2014, vol. 7, 222-229
- **LIU et al.** *Process Biochemistry*, 2018, vol. 71, 18-22
- **BERNANDO et al.** *Brazilian Journal of microbiology*, 2019, vol. 47
- **TURNER'S et al.** *Journal of Dairy Science*, 2017, vol. 100 (1), 124-128
- **JUODEIKIENE et al.** *LWT-Food Science and Technology*, 2016, vol. 72, 399-406
- *Current Limitions and Challenges with Lactic Acid Bacteria: A review Saeed A. Hayek, Salam A. Ibrahim Food & Nutritional Sciences Program*
- **DUBOIS M** ; **GILLES K.A.** ; **HAMILTON J.K.** ; **REBERS P.A.** ; **SMITH F.** *Colorimetric Method for Determination of Sugar and Related Substances, Division of Biochemist*, https://ediscipiscinas.usp.br/pluginfile.php/5012807/mod resource/content/0/-dubois 1956%20%28m%C3%A9todo%29. pdf]
- **BORSHCHEVSKAYA L. N** ; **GORDEEVA T. L** ; **KALININA A.N.** ; **SINEOKII S.P.** Spectrophotometric Determination of Lactic Acid. *State Research Institute of Genetics and Selection of Industrial Microorganisms*, 2016